(19) Europäisches Patentamt
European Patent Office
Office européen des brevets

(11) EP 0 695 287 B1

(12) # EUROPÄISCHE PATENTSCHRIFT

(45) Veröffentlichungstag und Bekanntmachung des Hinweises auf die Patenterteilung:
**29.10.1997 Patentblatt 1997/44**

(21) Anmeldenummer: **94913120.5**

(22) Anmeldetag: **07.04.1994**

(51) Int Cl.6: **C07C 69/616**, C07F 9/40, C07D 319/06, C07C 57/26, C07C 69/738, C07C 49/792, C07C 205/57, C07D 317/24, C07D 273/01, C07C 205/43, C07C 49/83

(86) Internationale Anmeldenummer:
**PCT/EP94/01079**

(87) Internationale Veröffentlichungsnummer:
**WO 94/25424 (10.11.1994 Gazette 1994/25)**

(54) **FULLERENDERIVATE, VERFAHREN ZUR HERSTELLUNG UND DEREN VERWENDUNG**

FULLERENE DERIVATIVES, METHODS OF PREPARING THEM AND THEIR USE

DERIVES FULLERENES, LEUR PROCEDE DE FABRICATION ET LEUR UTILISATION

(84) Benannte Vertragsstaaten:
**BE CH DE FR GB IT LI NL**

(30) Priorität: **24.04.1993 DE 4313481**

(43) Veröffentlichungstag der Anmeldung:
**07.02.1996 Patentblatt 1996/06**

(73) Patentinhaber: **HOECHST AKTIENGESELLSCHAFT**
**65926 Frankfurt am Main (DE)**

(72) Erfinder: **BINGEL, Carsten**
**D-65929 Frankfurt (DE)**

(56) Entgegenhaltungen:
- **CHEMISCHE BERICHTE Bd. 126 , 3. Mai 1993 , WEINHEIM DE Seiten 1957 - 1959 BINGEL C 'CYCLOPROPANIERUNG VON FULLERENEN'**

EP 0 695 287 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

**Beschreibung**

Fullerene sind käfigförmige Kohlenstoffallotrope der allgemeinen Formel ($C_{20+2m}$) (mit m= natürliche Zahl). Sie enthalten zwölf Fünf- sowie beliebig viele, mindestens aber zwei Sechsringe aus Kohlenstoffatomen. Obwohl diese Verbindungsklasse erst 1985 von Kroto und Smalley nachgewiesen (Nature, 1985, 318, 162) wurde und Krätschmer und Huffman erst 1990 über die Darstellung makroskopischer Mengen an $C_{60}$ berichteten (Nature 1990, 347, 354), sind solche Verbindungen sehr schnell auf ein breites Interesse gestoßen und wurden innerhalb kürzester Zeit Gegenstand zahlreicher Forschungsarbeiten (siehe z.B. G.S. Hammond, V.J. Kuck (Editors), Fullerenes, American Chemical Society, Washington DC 1992 and Accounts of Chemical Research, Märzausgabe 1992).

Da man ein hohes Potential dieser Stoffklasse, beispielsweise im Bereich der Optoelektronik und Wirkstofforschung erwartet, wurden bereits Anstrengungen zur Derivatisierung, insbesondere von $C_{60}$, unternommen (siehe z. B. H. Schwarz, Angew. Chem. 1992, 104, 301 und F. Wudl et al. in G.S. Hammond, V.J. Kuck (Editors), Fullerenes, American Chemical Society, Washington DC 1992 und Accounts of Chemical Research, Märzausgabe 1992).

In einigen Derivatisierungsversuchen gelang es, definierte Produkte zu isolieren. Beispiele hierfür sind die Umsetzungen von Fullerenen in 1.3 dipolaren Cycloadditionen mit Diazoverbindungen (z.B. F. Wudl et al., Acc. Chem. Res. 1992, 25, 157) sowie in [2+1] Carbenadditionen mit nucleophilen Glycosylidencarbenen (z.B. A. Vasella et al., Angew. Chem. 1992, 104, 1383).

Weitere Beispiele sind die Addition von Nucleophilen, wie z.B. Lithium- und Magnesiumorganylen (z.B. A. Hirsch et al., Angew. Chem. 1992, 104, 808).

Wünschenswert war es, Fullerenderivate zu synthetisieren, die Struktureinheiten mit solchen funktionellen Gruppen enthalten, die bekanntermaßen Anwendung im Bereich der Wirkstofforschung finden, sich auch zum Aufbau neuer polymerer Materialien nutzen lassen und die physikalische Eigenschaften, wie z.B. Löslichkeit oder Polarität der Fullerenderivate, verbessern.

Es ist bereits seit langem bekannt, daß sich 1,3-Dicarbonylverbindungen wie Malonester und β-Ketoester bei der Synthese von Wirkstoffen bewährt haben (z.B. Organikum 16, beab. Aufl. 1986, S. 393, 413, 414).

Die Verknüpfung von C-H aciden Verbindungen, wie z.B. Malonestern oder β-Ketoestern mit aktivierten Olefinen ist als Michael-Addition bekannt (z.B. Organikum, 16., beab. Aufl. 1986, S 507). Es wurde nun gefunden, daß sich wohldefinierte Fullerenderivate erhalten lassen, indem Fullerene beispielsweise mit den Anionen von 2-Halocarbonylverbindungen umgesetzt werden.

Gegenstand der Erfindung ist ein Fullerenderivat der Formel I,

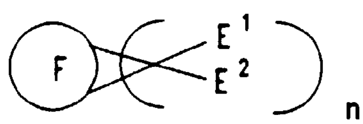

in der die Symbole und Indices die folgende Bedeutung haben:

F: ein Fullerenrest der Formel $C_{(20+2m)}$ mit m = 20, 25, 28, 29;

$E^1$, $E^2$: gleich oder verschieden COOH, COOR, $CONRR^1$, CHO, COR, CN, $P(O)(OR)_2$ und $SO_2R$, wobei R, $R^1$ einen geradkettigen oder verzweigten, gegebenenfalls ein- oder mehrfach gleich oder verschieden substituierten aliphatischen Rest ($C_1$ bis $C_{20}$) darstellen, in dem bis zu jede dritte $CH_2$-Einheit durch O oder $NR^4$ ersetzt sein kann, mit $R^4$ = ($C_1$-$C_{20}$)-Alkyl oder Benzyl, oder einen Benzylrest oder Phenylrest bedeuten, die gegebenenfalls durch 1 bis 5 Substituenten R, OH, OR, COOR, OCOR, $SO_3H$, $SO_2Cl$, F, Cl, Br, $NO_2$ und CN substituiert sein können

oder gleich

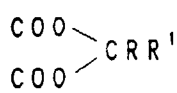

oder verschieden voneinander COR, R, oder H,

oder verschieden voneinander COR/R oder F/Cl/Br, wobei R die obengenannte Bedeutung hat, oder verschieden voneinander $NO_2$, $R^3$ oder H, wobei $R^3$ einen gegebenenfalls ein- oder mehrfach substituierten aliphatischen Rest ($C_1$ bis $C_{20}$) darstellt;

n: eine natürliche Zahl von 1 bis 10+m mit m= 20, 25, 28, 29.

Bevorzugt sind Verbindungen der Formel I, in der die Symbole und Indices die folgende Bedeutung haben:

F: ein Fullerenrest der Formel $C_{(20+2m)}$ mit m = 20, 25, 28, 29,

$E^1$, $E^2$: gleich oder verschieden COOR, COR, $P(O)(OR)_2$, COOH, CN, wobei R einen geradkettigen oder verzweigten, gegebenenfalls ein- oder mehrfach gleich oder verschieden substituierten aliphatischen Rest ($C_1$ bis $C_{20}$) darstellt, in dem bis zu jede dritte $CH_2$-Einheit durch O oder $NR^4$ ersetzt sein kann, mit $R^4$ = ($C_1$-$C_{20}$)-Alkyl oder Benzyl, oder einen Benzylrest oder Phenylrest bedeutet, die gegebenenfalls durch 1 bis 3 Substituenten R, OH, OR, COOR, OCOR, $SO_3H$, $SO_2Cl$, F, Cl, Br, $NO_2$ und CN substituiert sein können,

oder gleich

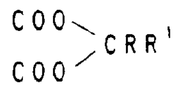

oder verschieden voneinander COR, R oder H,
oder verschieden voneinander COR/R oder F/Cl/Br,

n: eine natürliche Zahl von 1-12.

Besonders bevorzugt sind Verbindungen der Formel I, in der die Symbole und Indices die folgende Bedeutung haben:

F : $C_{60}$, $C_{70}$

E1/E2: $CO_2R^1$ / $CO_2R^2$ ;
$CO_2R^1$/$COR^2$ ;
$CO_2R^1$/CN ;
COAr / $R^1$ oder H;
COAr / $R^1$ oder Cl;

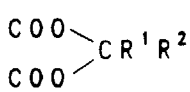 ;

$COR^1$/$COR^2$;
$P(O)(OR^1)_2$ / $P(O)(OR^2)_2$;
COOH/COOH;
wobei $R^1$ und $R^2$, gleich oder verschieden, einen geradkettigen oder verzweigten, gegebenenfalls ein- oder mehrfach gleich oder verschieden substituierten Alkylrest ($C_1$ bis $C_{20}$) darstellen, in dem bis zu jede dritte $CH_2$-EinheEt durch O oder $NR^4$ ersetzt sein kann, mit $R^4$ = ($C_1$-$C_{20}$)-Alkyl oder Benzyl, oder einen Benzylrest oder Phenylrest bedeutet, die gegebenenfalls durch 1 bis 3 Substituenten OH, OMe, $CO_2R^1$, $OOCR^1$, $SO_3H$, $SO_2Cl$, F, Cl, Br, $NO_2$ und CN substituiert sein können, und Ar einen Phenylrest darstellt, der ebenfalls durch 1 bis 3 Substituenten OH, OMe, Me, $CO_2R^1$, $OCOR^1$, $SO_3H$, $SO_2Cl$, F, Cl, Br, $NO_2$ und CN substituiert sein kann oder durch einen geradkettigen oder verzweigten, gegebenenfalls ein- oder zweifach gleich oder verschieden durch $COOR^5$, $CONHR^5$, $CONR^5_2$, $CONH_2$, $CONR^6$, COOH, OH oder $OCOR^5$, COOAr, $COOCH_2Ar$ substituierten aliphatischen Rest ($C_1$-$C_{20}$), vorzugsweise $C_1$-$C_{10}$, substituiert sein kann
mit $R^5$ =

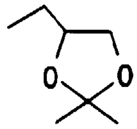 , 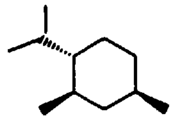 ,

$C_1$-$C_6$-Alkyl, Hydroxy-($C_1$-$C_6$)-Alkyl, Carboxyl($C_1$-$C_6$)-alkyl oder ($C_1$-$C_3$)-Alkylcarboxyl($C_1$-$C_6$)-alkyl;

$R^6$= $C_{11}$-$C_{17}$-Alkylen, in dem bis zu jede 3. $CH_2$-EinheEt durch O ersetzt sein kann, das gemeinsam mit dem Amidstickstoff einen $C_{12}$-$C_{18}$Ring bildet, und Ar wie oben definiert;

n : eine natürliche Zahl von 1-6 bedeutet.

Ganz besonders bevorzugt sind Verbindungen der Formel I, in denen die Symbole und Indices die folgende Bedeutung haben:

F : $C_{60}$, $C_{70}$

$E^1/E^2$: $CO_2Alkyl^1/CO_2Alkyl^1$ ;
$CO_2Alkyl^1/COAlkyl^2$ ;
COAr/Ar ;
$COAr/Alkyl^1$ ;
COAr / H
wobei $Alkyl^1$, $Alkyl^2$ einen geradkettigen oder verzweigten Alkylrest mit 1 bis 10 C-Atomen darstellt, in dem bis zu jede dritte $CH_2$-Einheit durch O ersetzt sein kann, und Ar eine Phenylgruppe bedeutet, die durch einen geradkettigen oder verzweigten, gegebenenfalls ein- oder zweifach gleich oder verschieden durch $COOR^5$, $CONHR^5$, $CONR^5_2$, $CONR^6$, COOH, OH oder $OCOR^5$ substituierten aliphatischen Rest ($C_1$-$C_6$) substituiert sein kann, worin $R^5$ und $R^6$ die obengenannte Bedeutung haben,

n : 1 oder 2.

Der geradkettige oder verzweigte aliphatische Rest ($C_1$-$C_{20}$) R, $R^1$ kann zum Beispiel, vorzugsweise ein- oder zweifach, gleich oder verschieden durch OH, COOH, COOAr, $CONR^5_2$, $COONR^6$, $OCOR^5$, $COOCH_2Ar$, $CONHCH_2Ar$, CONHAr, $CONHR^5$, $COOR^5$, Halogen, $CONH_2$, $COCH_2Ar$, COAr, $CO(C_1$-$C_6)$-Alkyl oder CHO substituiert sein, worin Ar, $R^5$ und $R^6$ die obengenannte Bedeutung haben.

Die erfindungsgemäßen Verbindungen der Formel I werden beispielsweise durch Cyclopropanierung von Fulleren mit einer α-Halo-CH-aciden Verbindung in Gegenwart einer geeigneten Base dargestellt (z.B. L.L. McCoy, J. Amer. Chem. Soc. 1958, 80, 6568) oder durch Umsetzung geeignet funktionalisierter cyclopropanierter Fullerenderivate nach bekannten Methoden, wobei zu beachten ist, daß die eingesetzten Reagenzien nicht mit dem elektrophilen Fullerenrest reagieren.

Weiterer Gegenstand der Erfindung ist das folgende allgemeine Verfahren zur Herstellung von Fullerenderivaten der Formel I

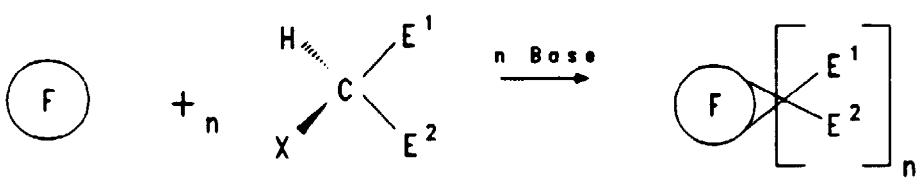


in dem

F einen Fullerenrest der Formel $C_{(20+2m)}$ mit m = 20, 25, 28, 29,

$E^1$ und $E^2$ gleich oder verschieden COOH, COOR, $CONRR^1$, CHO, COR, CN, $P(O)(OR)_2$ oder $SO_2R$, wobei R, $R^1$

einen geradkettigen oder verzweigten, gegebenenfalls ein- oder mehrfach gleich oder verschieden substituierten aliphatischen Rest ($C_1$ bis $C_{20}$) darstellt, in dem bis zu jede dritte $CH_2$-Einheit durch O oder $NR^4$ ersetzt sein kann, mit $R^4$ = ($C_1$-$C_{20}$)-Alkyl oder Benzyl, oder einen Benzylrest oder Phenylrest bedeutet, gegebenenfalls durch 1 bis 5 Substitutenten R, OH, OR, COOR, OCOR, $SO_3H$, $SO_2Cl$, F, Cl, Br, $NO_2$ und CN substituiert sein können,

oder gleich

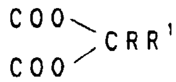 ,

oder verschieden voneinander COR, R oder H,
oder verschieden voneinander COR/R oder F/Cl/Br,
oder verschieden voneinander $NO_2$, $R^3$ oder H, wobei $R^3$ ein gegebenenfalls ein- oder mehrfach substituierter aliphatischer Rest ($C_1$ bis $C_{20}$) sein kann,

| | |
|---|---|
| X | -Cl, -Br, -I, $-OSO_2Ar$, $OSO_2CF_3$, $OSO_2C_4F_9$ |
| Base: | Alkalimetallhydride, Alkalimetallhydroxide, Alkoholate, Amide, Amine, Guanidine |
| n | eine natürliche Zahl von 1 bis 10+m mit m = 20, 25, 28, 29 |

bedeutet.

Bevorzugt ist ein Verfahren zur Herstellung von Verbindungen der Formel I, bei dem ein Fulleren der allgemeinen Formel $C_{(20+2m)}$ (m = 20, 25, 28, 29) in einem aprotischen organischen Lösungsmittel wie Toluol, Chlorbenzol, Benzol, $CH_2Cl_2$ mit Verbindungen der Formel II in Gegenwart geeigneter Basen in einem Temperaturbereich von -78°C bis 180°C, vorzugsweise 0 bis 110°C und in geeigneten Fällen bei Raumtemperatur (20-30°C) umgesetzt wird.

Die Wahl der Base richtet sich nach dem pKa-Wert und der Empfindlichkeit der C-H aciden Verbindung gegenüber der eingesetzten Base.

Zur Herstellung von Verbindungen der Formel I mit n = 1 wird bei annähender Stöchiometrie der Ausgangsverbindungen vorzugsweise in einem Temperaturbereich von -78°C bis +50°C, besonders bevorzugt bei 0°C bis 50°C gearbeitet. Ein hoher Substitutionsgrad und damit ein großer Wert für n wird erreicht, indem überschüssige CH-acide Verbindung der Formel II und hinreichende Menge an Base eingesetzt und zur Reaktionsbeschleunigung gegebenenfalls auf über 100°C erhitzt wird.

Die nach dem erfinderischen Verfahren erhältlichen Verbindungen der Formel I lassen sich aber auch durch Folgereaktionen wohldefiniert herstellen in dem zum Beispiel ein Ester der Formel I zur entsprechenden Säure der Formel I verseift wird oder ein Alkohol der Formel I mit einer Säure zu einem Ester der Formel I umgesetzt wird oder ein Ester der Formel I mit einem Amin zu dem entsprechenden Amid der Formel I umgesetzt wird.

Als Fullerene werden bevorzugt reines $C_{60}$ und/oder $C_{70}$ eingesetzt aber auch Rohfullerene, die ein Gemisch aus $C_{60}$ und $C_{70}$ als Hauptkomponenten enthalten. Es können aber auch alle anderen denkbaren Fullerene bzw. Fullerenderivate eingesetzt werden.

Die Fullerene können durch Herstellung von Fullerenruß im Lichtbogenverfahren mit anschließender Extraktion mit einem unpolaren organischen Lösungsmittel (Rohfulleren), wie z.B. in WO 92/09279 beschrieben, gewonnen werden. Die weitere Feinauftrennung kann säulenchromatographisch erfolgen.

Die eingesetzten Fullerene sind zum Teil auch Handelsprodukte.

Als Cyclopropanierungsreagenzien können einerseits käufliche α-Halo-CH-acide Verbindungen eingesetzt werden oder man erhält die verwendeten Verbindungen der Formel II nach dem Chemiker bekannten Verfahren,wie der Halogenierung von CH-aciden Verbindungen oder beispielsweise der Friedel-Crafts-Acylierung von substituierten Aromaten mit Bromacetylhalogeniden. Ester- und Amidfunktionen werden aus den gewünschten Carbonsäuren und Alkoholen bzw. Aminen nach bekannten Verfahren erhalten.

Die erfindungsgemäßen Verbindungen der Formel I finden beispielsweise Anwendung in optoelektronischen Bauelementen.

Die Erfindung wird durch die Beispiele näher erläutert.

Abkürzungsverzeichnis

| | |
|---|---|
| $CDCl_3$ : | Deuterotrichlormethan |

| | |
|---|---|
| $CD_2Cl_2$ : | Dideuterodichlormethan |
| $CS_2$ : | Schwefelkohlenstoff |
| DBU : | 1,8-Diazobicyclo[5.4.0]undec-7-en |
| DMAP : | 4-Dimethylaminopyridin |
| d : | Duplett (NMR) oder Tag(e) (Zeitangabe) |
| $Et_2O$ : | Diethylether |
| HPLC : | Hochdruckflüssigkeitschromatographie |
| h : | Stunden |
| MS (FAB) : | Massenspektroskopie (Fast-Atom-Bombardment) |
| m : | medium (IR) oder Multiplett (NMR) |
| NaH : | Natriumhydrid |
| q oder quart : | Quartett (NMR) |
| quint : | Quintett (NMR) |
| $R_f$ : | Ratio of Fronts in der Dünnschichtchromatographie |
| S : | Singulett (NMR) oder strong (IR) |
| $SiO_2$ : | Kieselgel für chromatographische Zwecke |
| t : | Triplett (NMR) |
| w : | weak (IR) |

Beispiel 1

In einem 250 ml Stickstoffkolben wurden 435 mg (0.60 mmol) $C_{60}$ in 200 ml Toluol gelöst. Es wurden 0.144 g (6.0 mmol) NaH zugegeben und 0.216 g (0.90 mmol) Brommalonsäurediethylester hinzugefügt. Die Suspension wurde 6.5 h bei Raumtemperatur gerührt, mit 8 Tropfen 1 molarer $H_2SO_4$-Lösung gequencht mit Magnesiumsulfat getrocknet und filtriert. Nach HPLC wurden 65 % des eingesetzten $C_{60}$ umgesetzt. Nach Chromatographie über Kieselgel (0.063 - 0.2 mm) mit Toluol/Hexan 1:1 und Toluol wurde

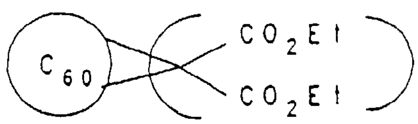
1

in mikrokristalliner Form erhalten (0.238 g, 45 %).

$R_f(SiO_2$; Toluol) = 0.50
MS(FAB) : 878 ($M^+$)
IR (auf KBr): $\nu$ [$cm^{-1}$] = 2979(w), 1745(C=O), 1428($C_{60}$), 1295(m), 1266(m), 1234(s), 1206(m), 1186($C_{60}$), 1095 (m), 1061(w).
$^1$H-NMR (360 MHz, $CDCl_3$): $\delta$= 4.57 (q, J= 7.13 Hz, 4H), 1.49 (t, J= 7.13 Hz, 6H).
$^{13}$C-NMR (100 MHz, $CDCl_3$) $\delta$= 163.55, 145.35, 145.26, 145.20, 145.18, 144.88, 144.69, 144.67, 144.60, 143.88, 143.08, 143.01, 142.99, 142.21, 141.92, 140.94, 139.03, 71.64, 63.37, 52.26, 14.22.

| | | | |
|---|---|---|---|
| Analyse | ber. für $C_{67}H_{10}O_4$: | C: 91.6 % | H: 1.1 % |
| | gef. | C: 92.4 % | H: 1.4 % |

Beispiel 2

Analog zu Beispiel 1 wurde bei der Umsetzung von 0.7 g (0.98 mmol) $C_{60}$, 0.29 g (1.2 mmol) Brommalonsäurediethylester und 0.23 g (9.6 mmol) NaH nach Aufarbeitung und Chromatographie an Kieselgel (0.063-0.2 mm) neben 0.33 g (39 %) Monoaddukt auch noch

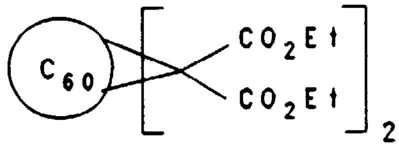

isoliert (0.048 g, 4.7 %). Laut DC mehr als 1 Isomer

$R_f(SiO_2$; Toluol) = 0.19 bis 0.24
MS(FAB) : 1036 ($M^+$)

Beispiel 3

Unter Schutzgas wurden 471 mg (0.654 mmol) $C_{60}$ in 200 ml Toluol mit 207 mg (0.981 mmol) Brommalonsäuredimethylester und 125 mg (0.821 mmol) DBU versetzt und 4 h bei Raumtemperatur gerührt. Das Reaktionsgemisch wurde filtriert und die Lösung auf 60 ml Volumen eingeengt.
Laut HPLC wurden 69 % $C_{60}$ umgesetzt. Nach Chromatographie an $SiO_2$ (0.063-0.2 mm) mit Toluol/i-Hexan 1:1, 2:1 und 3:1 wurde

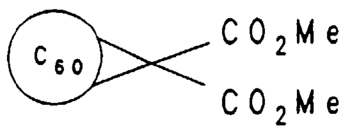

in mikrokristalliner Form erhalten (249.3 mg, 44 %) und daneben 114 mg (24 %) $C_{60}$ zurückgewonnen.

$R_f(SiO_2$; Toluol) = 0.37 (Toluol)
MS(FAB): 850 ($M^-$)
$^1$H-NMR (360 MHz, $CDCl_3/CS_2$): $\delta$ = 4.06 (s).

Beispiel 4

Analog zu Beispiel 3 wurden 0.137 g (0.190 mmol) $C_{60}$ in 58 ml Toluol mit 88 mg (0.19 mmol) Brommalonsäuredidecylester und 29 mg (0.19 mmol) DBU umgesetzt und 15 h bei Raumtemperatur gerührt. Laut HPLC wurden 75 % $C_{60}$ umgesetzt. Das Reaktionsgemisch wurde völlig eingeengt, der Rückstand mit Diethylether extrahiert (3 x 10 ml), die Etherlösung durch Kieselgel filtriert und nach Entfernen des Ethers an $SiO_2$ (0.063-0.2 mm) mit Toluol/i-Hexan 1:1 und 2:1 chromatographiert.

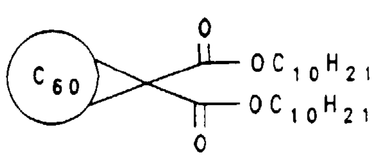

wurde als braunes zähes Öl gewonnen (66 mg, 31 %).

$R_f(SiO_2$; Toluol/i-Hexan 1:1) = 0.4
MS(FAB): 1102 ($M^-$).
$^1$H-NMR (360 MHz, $CDCl_3$): $\delta$ = 4.47 (t, J= 6.5 Hz, 4H), 1.82 (m, 4H), 1.43 (m, 4H), 1.26 (m, 24H), 0.82 (t, J= 6.0 Hz, 6H).
$^{13}$C-NMR (100 MHz, $CDCl_3$): $\delta$ = 163.65, 145.39, 145.24, 145.17, 145.16, 144.86, 144.67, 144.65, 144.59, 143.87, 143.06, 143.00, 142.97, 142.19, 141.90, 140.93, 138.99, 71.70, 67.46, 52.47, 31.88, 29.61, 29.56, 29.33, 29.24,

28.61, 25.99, 22.68, 14.12

Beispiel 5

Analog zu Beispiel 3 wurden 943 mg (1.31 mmol) $C_{60}$ in 400 ml Toluol mit 506 mg (1.31 mmol) Brommalonsäuredi-(2-(2-Methoxyethoxy)-ethyl)ester und 199 mg (1.31 mmol) DBU umgesetzt. Nach 18 h Reaktionszeit bei Raumtemperatur wurde der Reaktionsansatz filtriert und an 50 g Kieselgel (0.063-0.2 mm) chromatographiert. $C_{60}$ wurde mit Toluol eluiert und das Monoadditionsprodukt mit 800 ml Toluol/Diethylether 1:1 eluiert. Es wurden 504 mg (37 % bezogen auf eingesetztes $C_{60}$) Monoadditionsprodukt

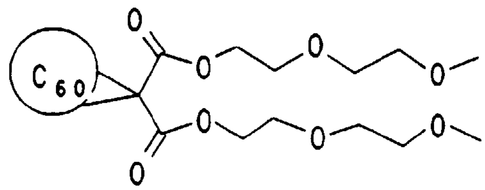

erhalten.

$R_f(SiO_2$; Toluol/$Et_2O$ 1:1) = 0.21
MS(FAB): 1026 ($M^-$, 40 %), 720 (100 %)
$^1$H-NMR (360 MHz, $CDCl_3$): $\delta$ = 4.64 (m, 4H), 3.87 (m, 4H), 3.67 (m, 4H), 3.53 (m, 4H), 3.36 (s, 6H).

Beispiel 6

Analog zu Beispiel 3 wurden 236 mg (0.33 mmol) $C_{60}$ in 100 ml Toluol mit 50 mg (0.33 mmol) DBU und einer Lösung von 74 mg (0.33 mmol) 2,2-Dimethyl-5-bromo-4,6-diketo-1,3-dioxan in 2 ml Methylenchlorid umgesetzt. Nach 18 h Reaktionszeit bei Raumtemperatur wurde der Reaktionsansatz filtriert, auf das halbe Volumen eingeengt und am 100 g Kieselgel (0.063-0.2 mm) chromatographiert. Mit 300 ml Toluol/i-Hexan 1:1 wurde $C_{60}$ mit 350 ml Toluol das Monoadditionsprodukt eluiert. Nach Entfernen des Lösungsmittels wurde das mikrokristalline schwarze Monoadditionsprodukt mit Pentan und Diethylether gewaschen und im Vakuum getrocknet. Es wurden 60.2 mg (21 %)

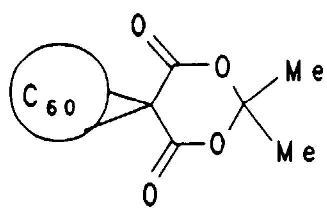

erhalten.

$R_f(SiO_2$; Toluol) = 0.40
MS(FAB): 892 ($M^{\ominus}$, 20 %), 804 ($M^{\ominus}$-Aceton, 25 %), 760 ($M^{\ominus}$-Aceton-$CO_2$, 55 %), 720 (100 %).
$^1$H-NMR (360 MHz, $CDCl_3$): $\delta$ : 2.18 (s)
$^{13}$C-NMR (100 MHz, $CDCl_2$): $\delta$ = 160.11(C=O), 145.90, 145.66, 145.62(2C), 145.42, 145.15, 145.01(2C), 145.00, 144.40, 143.82, 143.57(2C), 143.50, 143.44, 142.64, 141.89, 141.75, 141.36, 106.34, 71.69, 44.74, 28.33

Beispiel 7

Analog zu Beispiel 3 wurden 123 mg (0.17 mmol) $C_{60}$ in 50 ml Toluol mit 51 mg (0.17 mmol) Brommalonsäureditert.-butyl-ester und 26 mg (0.17 mmol) DBU umgesetzt. Nach 4 h Reaktionszeit bei Raumtemperatur wurde der Reaktionsansatz filtriert, auf ein Volumen von 20 ml eingeengt und an 50 g Kieselgel (0.063-0.2 mm) chromatographiert. Mit dem Laufmittelgemisch Toluol/i-Hexan 1:1.5 wurde $C_{60}$ und das Monoadditionsprodukt eluiert und von den Mehrfachadditionsprodukten abgetrennt. Nach Entfernen des Lösungsmittels wurde der Rückstand in Methylenchlorid aufgenommen, wobei nur geringe Mengen $C_{60}$ neben dem Monoadditionsprodukt extrahiert wurden, und die Methylenchloridlösung wurde auf 5 ml eingeengt. Die chromatographische Trennung an 80 g Kieselgel (0.063-0.2mm) mit Toluol/i-Hexan 1:2 und Toluol ergab nach Waschen mit Pentan und Diethylether und Trocknen im Vakuum 60 mg (37 %) Monoadditionaddukt.

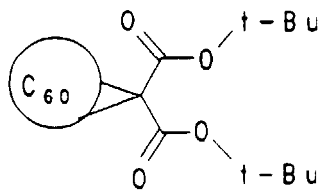

$R_f(SiO_2$; Toluol) = 0.58
MS(FAB): 934 ($M^{\ominus}$, 75 %), 734 (90 %), 720 (100 %).
$^{1}$H-NMR (360 MHz, $CDCl_3$) : $\delta$ = 1.68 (s)

Beispiel 8

In einem 100 ml Zweihalskolben wurden unter Argonatmosphäre 50 mg (0.054 mmol) Bis-(t-butoxycarbonyll-methano-fulleren aus Beispiel 7 in 30 ml Chloroform gelöst und mit 650 mg (6.7 mmol) Methansulfonsäure versetzt. Nach etwa 2 h bildete sich ein Niederschlag. Nach 24 h war die über dem Niederschlag stehende Lösung farblos. Der Niederschlag wurde vom Lösungsmittel abgetrennt und zweimal mit Ether gewaschen, um überschüssige Säure zu entfernen. Nach dem Trocknen im Vakuum wurden 19 mg (43 %)

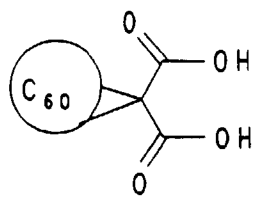

erhalten.
MS(FAB): 822 ($M^{\ominus}$, 30 %), 778 ($M^{\ominus}$-$CO_2$, 30 %), 734 ($M^{\ominus}$-2 $CO_2$, 100 %), 720 ( 70 %).

Beispiel 9

In einem 250 ml Stickstoffkolben wurden 236 mg (0.328 mmol) Fulleren $C_{60}$ in 100 ml Toluol vorgelegt und 102 mg (0.67 mmol) DBU sowie 62 mg (0.412 mmol) 2-Chloracetessigsäuremethylester zugespritzt. Nach 2 h wurde das Reaktionsgemisch filtriert.

Laut HPLC wurden 80 % des eingesetzten $C_{60}$ umgesetzt. Nach Chromatographie an Kieselgel (0.063-0.2 mm) mit Toluol/i-Hexan (1:1) und Toluol wurde

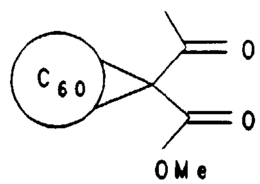

in mikrokristalliner Form erhalten (75 mg, 27 %).

$R_f(SiO_2$; Toluol) = 0.44
MS(FAB) : 834 ($M^-$)
IR (auf KBr): $v$ [$cm^{-1}$] = 2996(w), 2943(w), 1756(C=O), 1718(C=O), 1428($C_{60}$), 1356(w), 1265(w), 1231(s), 1200 (m), 1186($C_{60}$).
$^1$H-NMR (360 MHz, $CDCl_3$): $\delta$ = 4.10 (s, 3H), 2.87 (s, 3H).
$^{13}$C-NMR (100 MHz, $CDCl_3$): $\delta$ = 193.64, 164.45, 145.44, 145.28, 145.28, 145.25, 145.21, 145.20; 145.08, 144.87, 144.75, 144.75, 144.74, 144.74, 144.69, 144.61, 144.61, 143.88, 143.84, 143.17, 143.12, 143.07, 143.06, 143.06, 142.98, 142.24, 142.24, 141.93, 141.90, 141.08, 141.01, 139.45, 138.03, 72.33, 54.09, kein Signal für Methoxy-C, 28.84.

| | | | |
|---|---|---|---|
| Analyse | ber. für $C_{65}H_6O_3$: | C: 93.53 | H: 0.72 |
| | gef. | C: 93.4 | H: 0.8 |

Beispiel 10

Analog zu Beispiel 9 wurden 0.471 g (0.654 mmol) $C_{60}$ in 200 ml Toluol mit 108 mg (0.656 mmol) 2-Chloracetessigsäureethylester und 99.5 mg (0.654 mmol) DBU umgesetzt und 2.5 h bei Raumtemperatur gerührt. Das Reaktionsgemisch wurde filtriert und die Lösung auf 60 bis 70 ml eingeengt. Laut HPLC wurden nur 24 % $C_{60}$ umgesetzt. Nach zweimaliger Chromatographie an $SiO_2$ (0.063-0.2 mm) mit Toluol/i-Hexan 1:1 und Toluol wurde

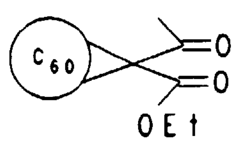

in mikrokristalliner Form erhalten (111.5 mg, 20 %) und daneben 280 mg (59 %) $C_{60}$ zurückgewonnen.

$R_f(SiO_2$; Toluol) = 0.36
MS(FAB): 848 ($M^-$)
$^1$H-NMR (360 MHz, $CDCl_3$): $\delta$ = 4.55 (q, J = 7.1 Hz, 2H), 2.82 (s, 3H), 1.54 (t, J = 7.1 Hz, 3H).

Beispiel 11

In einem 250 ml 2-Halskolben wurden 151 mg (0.65 mmol) Desylchlorid und 367 mg (3.27 mmol) Kaliumtertiärbutanolat vorgelegt und anschließend eine Lösung von 236 mg (0.328 mmol) $C_{60}$ in 100 ml Toluol zugegeben. Es wurde 40 h bei Raumtemperatur gerührt, durch Zugabe von 5 Tropfen 1 molarer $H_2SO_4$-Lösung gequencht, mit $MgSO_4$ getrocknet und filtriert. Laut HPLC wurden 60 % des eingesetzten $C_{60}$ umgesetzt. Nach Chromatographie an Kieselgel (0.063 - 0.2 mm) mit Toluol/i-Hexan 2:3 und Toluol wurde

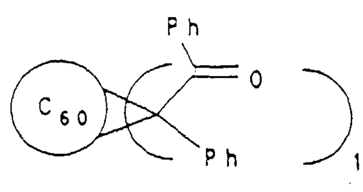

in mikrokristalliner Form erhalten (76 g, 25 %).

$R_f(SiO_2$, Toluol/i-Hexan 1:1) = 0.54
MS(FAB) : 914 ($M^-$)
IR (auf KBr): $v$ [$cm^{-1}$] = 3051(w), 3036(w), 1678(C=O), 1595(m), 1494(w), 1444(m), 1427($C_{60}$), 1255(m), 1187

($C_{60}$), 697(s).
$^1H$-NMR (360 MHz, $CDCl_3$): δ = 8.62 (m, 2H), 8.24 (m, 2H), 7.62-7.40 (m, 5H).
$^{13}C$-NMR (100 MHz, $CDCl_3$) δ= 190.57, 148.20, 146.31, 145.59, 145.29, 145.28, 145.21, 145.19, 145.19, 144.83, 144.81, 144.77, 144.74, 144.60, 144.50, 144.41, 143.92, 143.77, 143.17, 143.05, 143.01, 142.99, 142.97, 142.88, 142.34, 142.19, 142.19, 142.13, 141.13, 141.04, 138.51, 137.19, 134.22, 133.55, 132.21, 132.16, 130.34, 129.08, 128.99, 128.97, 75.78, 60.81.

| | | | |
|---|---|---|---|
| Analyse | ber. für $C_{74}H_{10}O$:<br>gef. | C: 91.6 %<br>C: 92.4 % | H: 1.1 %<br>H: 1.4 % |

Beispiel 12

Analog zu Beispiel 3 wurden 471 mg (0.654 mmol) $C_{60}$ in 200 ml Toluol mit 100 mg (0.657 mmol) DBU und 140 mg (0.657 mmol) α-Brompropiophenon umgesetzt. Nach 8 Tagen Reaktionszeit bei Raumtemperatur haben sich laut HPLC 52 % des $C_{60}$ umgesetzt. Nach zweimaliger Chromatographie an $SiO_2$ (0.063-0.2 mm) mit Toiuol/i-Hexan 1:2 → 2:1 und 2:3/1:1 wurde

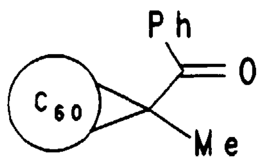

erhalten (30 mg, 5.4 %).

$R_f$($SiO_2$; Toluol/i-Hexan 1:1) = 0.31
MS(FAB): 852 ($M^-$).
$^1H$-NMR (360 MHz, $CDCl_3$): δ = 8.53 (m, 2 $H_{ortho}$), 7.70 (m, 1 $H_{para}$), 7.64 (m, 2 $H_{meta}$), 2.64 (s, 3H).

Beispiel 13

In einen 250 ml Stickstoffkolben wurden 0.236 g (0.328 mmol) $C_{60}$ in 100 ml Toluol gelöst. Es wurden 66 mg (0.331 mmol) ω-Bromacetophenon und 0.051 g (0.334 mmol) DBU zugegeben und bei Raumtemperatur gerührt. Nach 5 h wurden 2 Tropfen 2n Schwefelsäure zugegeben und das Reaktionsgemisch mit $MgSO_4$ getrocknet. Nach Filtration und Einengen der Lösung auf die Hälfte wurde an Kieselgel chromatographiert, nachdem zuvor der $C_{60}$-Umsatz auf 72 % mit HPLC bestimmt worden war. Nach Chromatograhie an $SiO_2$ (0.063-0.2 mm) mit Toluol/i-Hexan (2/3 → 2/1) und Toluol wurde

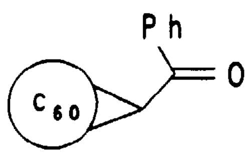

in mikrokristalliner Form erhalten (59 mg, 21 %), daneben noch 81 mg mit $C_{60}$ verunreinigtes Produkt.

$R_f$($SiO_2$, Toluol) = 0.64
MS(FAB) : 838 ($M^-$)
IR (auf KBr): $v$ [$cm^{-1}$] = 3023(w), 1684(C=0), 1446(m), 1428($C_{60}$), 1244(m), 1219(s), 1185($C_{60}$), 1006(s), 710(s), 683(s).
$^1H$-NMR (360 MHz, $CDCl_3$): δ = 8.46 (m, 2H), 7.75 (m, 1H), 7.67 (m, 2H), 5.64 (s, $J_{CH}$ = 162 Hz, 1H).
$^{13}C$-NMR (100 MHz, $CDCl_3$): δ = 189.69, 148.04, 146.63, 145.58, 145.39, 145.31, 145.21, 145.20, 145.09,

144.87,144.75, 144.69, 144.66, 144.65, 144.65, 144.37, 143.96, 143.71, 143.34, 143.18, 143.05, 142.99, 142.99, 142.80, 142.49, 142.27, 142.23, 142.11, 141.22, 140.98, 139.61, 136.65, 135.95, 134.48, 129.35, 128.94, 72.30, 44.16.

| | | | |
|---|---|---|---|
| Analyse | ber. für $C_{68}H_6O$ | C: 97.37 % | H: 0.72 % |
| | gef. | C: 97.7 % | H: 0.8 % |

Beispiel 14

Analog zu Beispiel 13 wurde in einem Ansatz aus 0.236 g (0.328mmol) $C_{60}$ in 100 ml Toluol, 114 mg (0.572 mmol) ω-Bromacetophenon und 95 mg (0.62 mmol) DBU neben 83 mg (30 %) Monoaddukt auch das Diaddukt, als Isomerengemisch,

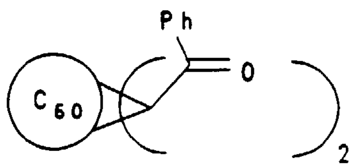

isoliert (54 mg, 17 %)

$R_f$ : 0.47 (Toluol)
MS(FAB) : 956 ($M^-$)

In den folgenden Beispielen wurden die Reaktionen in einer Argonatmosphäre durchgeführt, während die weitere Aufarbeitung der einzelnen Reaktionsansätze nicht unter Schutzgas erfolgte.
Unter Rühren wurde jeweils eine Lösung von $C_{60}$ in Toluol mit der CH-aciden Verbindung und DBU als Base bei Raumtemperatur umgesetzt. Nach Filtration der Reaktionsgemische und Einengen der Lösungen auf das halbe Volumen wurde an Kieselgel chromatographiert. Die isolierten Monoadditionsprodukte wurden mit Diethylether oder Pentan gewaschen und im Vakuum getrocknet.

Beispiel 15

236 mg (0.33 mmol) $C_{60}$ wurden in 100 ml Toluol mit 98 mg (0.33 mmol) 3-[4-(2-Bromacetyl)-phenyl]-propionsäureethylester und 50 mg (0.33 mmol) DBU für 4 Stunden gerührt.
Chromatographie: 75 g Kieselgel (0.063-0.2 mm); 400 ml Toluol 117 mg (38 %)

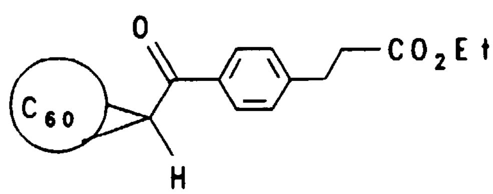

$R_f$ ($SiO_2$, Toluol) = 0.20
MS (FAB) : 938 ($M^{\ominus}$, 60 %), 720 (100 %)
$^1$H-NMR (360 MHz, $CHCl_3$): δ = 8.38 (d, J = 8.4 Hz, 2 H), 7.50 (d, J = 8.4 Hz, 2 H), 5.61 (s, 1 H), 4.14 (q, J = 7.1 Hz, 2 H), 3.10 (t, J = 7.6 Hz, 2 H), 2.71 (t, J = 7.6 Hz, 2 H), 1.24 (t, J = 7.1 Hz, 3 H)
$^{13}$C-NMR (100 MHz, $CDCl_3$): δ = 189.20, 172.37, 148.06, 147.99, 146.69, 145.58, 145.39, 145.28, 145.19, 145.18, 145.07, 144.86, 144.73, 144.67, 144.64, 144.62, 144.34, 143.94, 143.70, 143.32, 143.16, 143.04, 142.98, 142.97, 142.78, 142.48, 142.26, 142.21, 142.10, 141.20, 140.96, 139.57, 136.63, 134.16, 129.34, 129.25, 72.33, 60.68,

44.19, 35.21, 31.05, 15.5

Beispiel 16

247 mg (0.34 mmol) $C_{60}$ wurden in 100 ml Toluol mit 127 mg (0.34 mmol) 2-[4-(2-Bromacetyl)-benzyl]-malonsäure-diethylester und 52 mg (0.34 mmol) DBU für 3 Stunden gerührt.
Chromatographie: 70 g Kieselgel (0.063-0.2 mm); 1000 ml Toluol 139 mg (40 %)

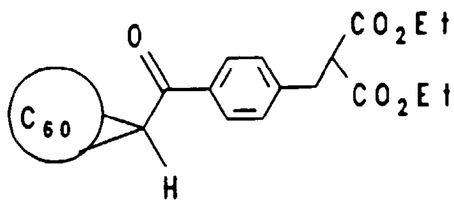

$R_f$ ($SiO_2$, Toluol) = 0.10
MS (FAB) : 1010 ($M^{\ominus}$ , 45 %), 720 (100 %)
$^1$H-NMR (360 MHz, $CD_2Cl_2$): $\delta$ = 8.40 (d, J = 8.4 Hz, 2 H), 7.54 (d, J = 8.4 Hz, 2 H), 5.71 (s, 1 H), 4.18 (m, 4 H), 3.75 (t, J = 7.7 Hz, 1 H), 3.36 (d, J = 7.8 Hz, 2 H), 1.23 (t, J = 7.1 Hz, 6 H).
$^{13}$C-NMR ($CD_2Cl_2$): $\delta$ = 189.1 (1C), 168.5, 148.3, 147.0, 145.7, 145.5, 145.5 (1C), 145.3, 145.2, 145.2, 145.1, 144.9 (1C), 144.8, 144.7, 144.7, 144.6, 144.4, 144.0, 143.8, 143.3, 143.2 (1C), 143.1, 143.0, 143.0, 142.9, 142.6, 142.3, 142.2, 142.2, 141.2, 141.0, 139.5, 136.8, 136.8, 134.6, 129.9, 129.2, 72.6, 61.8, 44.5, 34.7, 13.9.

Beispiel 17

123 mg (0.17 mmol) $C_{60}$ wurden in 50 ml Toluol mit 49 mg (0.17 mmol) 4-[4-(2-Bromacetyl)-phenyl]-buttersäure-ethylester und 26 mg (0.17 mmol) DBU für 15 Stunden gerührt.
Chromatographie: 40 g Kieselgel (0.063-0.2 mm); 300 ml Toluol 47 mg (28 %)

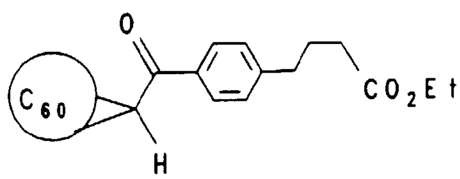

$R_f$ ($SiO_2$, Toluol) = 0.27
MS (FAB) : 952 ($M^{\ominus}$, 60 %), 720 (100 %)
$^1$H (360 MHz, $CD_2Cl_2$): $\delta$ = 8.41 (d, J = 8.3 Hz, 2 H), 7.52 (d, J = 8.3 Hz, 2H), 5.72 (s, 1 H), 4.13 (quart., J = 7.1 Hz, 2 H), 2.83 (t, J = 7.7 Hz, 2 H), 2.40 (t, J = 7.4 Hz, 2 H), 2.04 (quint, J = 7.6 Hz, 2 H), 1.26 (t, J = 7.1 Hz, 3H).

Beispiel 18

454 mg (0.63 mmol) $C_{60}$ wurden in 200 ml Toluol mit 350 mg (0.612 mmol) 3-[4-(2-Bromacetyl)-phenyl]-glutarsäure-di(4-nitrophenyl)ester auf etwa 50-60°C erwärmt und zusammen mit 93 mg (0.613 mmol) DBU für 15 Stunden gerührt.
Chromatographie: 50 g Kieselgel (0.063-0.2 mm); 500 ml Toluol, 600 ml Methylenchlorid
315 mg ($C_{60}$ (69 % bezogen auf eingesetztes $C_{60}$)
158 mg (20 % / 67 % bezogen auf eingesetztes/umgesetztes $C_{60}$)

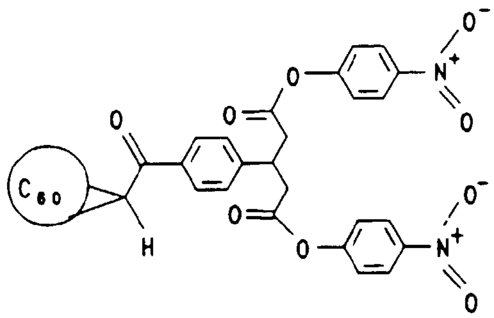

$R_f$ ($SiO_2$, $CH_2Cl_2$) = 0.30
MS (FAB) : 1210 ($M^{\ominus}$, 100 %), 720 (60 %)
$^1H$ (360 MHz, $CDCl_3$): $\delta$ = 8.49 (d, J = 8.3 Hz, 2 H), 8.21 (m, 4 H), 7.68 (d, J = 8.3 Hz, 2 H), 7.14 (m, 4 H), 5.61 (s, 1 H), 4.04 (quint, J = 7.4 Hz, 1 H), 3.22 (d, d, $J_{AB}$ = 16.2 Hz, $J_{AX}$ = 6.8 Hz, 2 H), 3.13 (d, d, $J_{AB}$ = 16.2 Hz, $J_{BX}$ = 8.1 Hz, 2 H)

Beispiel 19

371 mg (0.51 mmol) $C_{60}$ wurden in 150 ml Toluol mit 168 mg (0.44 mmol) 3-[4-(2-Bromacetyl)-phenyl]-propionsäure-(2,2-dimethyl-1,3-dioxolan-4-yl)-methylester und 67 mg (0.44 mmol) DBU für 15 Stunden gerührt.
Chromatographie: 25 g Kieselgel (0.063-0.2 mm); 100 ml Toluol, 102 ml Toluol/Ethanol 100:2, 205 ml Toluol/Ethanol 100:2.5
343 mg (76 % bezogen auf eingesetzte CH-acide Verbindung)

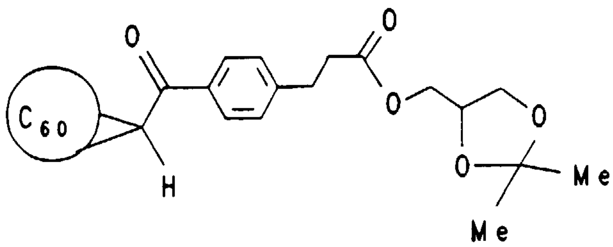

$R_f$ ($SiO_2$, Toluol/MeOH 20:1) = 0.68
MS (FAB) : I024 ($M^{\ominus}$, 50 %), 720 (100 %)
$^1H$-NMR (360 MHz, $CDCl_3$) $\delta$ = 8.38 (d, J = 8.3 Hz, 2 H), 7.50 (d, J = 8.3 Hz, 2 H), 5.61 (s, 1 H), 4.29 (m, 1 H), 4.18, 4.10 (m, 2 H), 4.05, 3.69 (m, 2 H), 3.11 (t, J = 7.5 Hz, 2 H), 2.78 (t, J = 7.5 Hz, 2 H), 1.43 (s, 3 H), 1.37 (s, 3 H)
$^{13}C$-NMR (100 MHz, $CDCl_3$) $\delta$ = 189.18 (C =O), 172.15 (C=O), 148.04, 147.69 (1C),146.66, 145.57, 145.38, 145.28, 145.18, 145.18, 145.07, 144.85 (1C), 144.73, 144.67, 144.64, 144.64, 144.62, 144.34, 143.93, 143.69, 143.32, 143.16 (1C), 143.03, 142.97 (1C), 142.97, 142.78, 142.47, 142.25, 142.21, 142.09, 141.20, 140.95, 139.58, 136.63, 134.24, 129.33, 129.28, 109.93, 73.56, 72.32, 66.26, 65.04, 44.16, 34.96, 30.92, 26.72, 25.37.

Beispiel 20

569 mg (0.79 mmol) $C_{60}$ wurden in 230 ml Toluol mit 299 mg (0.73 mmol) 3-[4-(2-Bromacetyl)-phenyl]-propionsäure und 113 mg (0.74 mmol) DBU für 24 Stunden gerührt.
Chromatographie: 150 g Kieselgel (0.063-0.2 mm); 400 ml Toluol/i-Hexan 1:1, 500 ml Toluol
186 mg $C_{60}$ (33 % bezogen auf eingesetztes $C_{60}$) zurückgewonnen
423 mg (55 % bezogen auf eingesetzte CH-acide Verbindung)

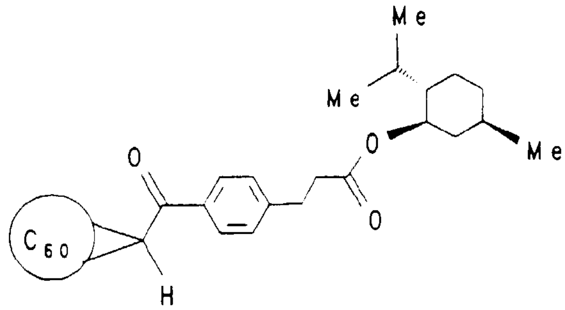

$R_f$ ($SiO_2$, Toluol) = 0.40
MS (FAB) : 1048 ($M^{\ominus}$ , 40 %), 720 (80 %)
$^1$H-NMR (360 MHz, $CD_2Cl_2$) $\delta$ = 8.39 (d, J = 8.3 Hz, 2 H), 7.53 (d, J = 8.3 Hz, 2 H), 5.70 (s, 1 H), 4.69 (m, 1 H), 3.11 (t, J = 7.5 Hz, 2 H), 2.72 (t, J = 7.5 Hz, 2 H), 1.94 (m, 1 H), 1.75 (m, 1 H), 1.67 (m, 2 H), 1.51-1.21 (m, 3 H), 1.11-0.8 (m, 2 H), 0.91 (d, J = 6.5 Hz, 3 H), 0.86 (d, J = 7.0 Hz, 3 H), 0.72 (d, J = 7.0 Hz, 3 H)
$^{13}$C-NMR (100 MHz, $CD_2Cl_2$) $\delta$ = 188.6 (C=0), 171.4 (C=0), 148.0, 147.9 (1C), 146.7, 145.3, 145.1, 144.9, 144.8, 144.8, 144.7, 144.5 (1C), 144.4, 144.3, 144.3, 144.2, 144.0, 143.6, 143.4, 142.9, 142.8 (1C), 142.7, 142.6, 142.6, 142.5, 142.2, 141.9, 141.8, 141.8, 140.8, 140.6, 139.2, 136.4 (1C), 136.4 (1C), 133.8 (1C), 129.0, 128.8, 74.0 (1C), 72.3 (2C), 46.8 (1C), 44.2, 40.6, 35.1, 33.9, 31.1, 30.8, 26.5, 23.1, 21.5, 20.3, 15.8

Beispiel 21

742 mg (1.03 mmol) $C_{60}$ wurden in 300 ml Toluol mit 350 mg (0.91 mmol) 4-[4-(2-Bromacetyl)-phenyl]-buttersäure-(6-hydroxyhexyl)-ester und 139 mg (0.91 mmol) DBU für 24 Stunden gerührt.
Chromatographie: 150 g Kieselgel (0.063-0.2 mm); 500 ml
Methylenchlorid/Methan 100:1 bis 100:3
359 mg $C_{60}$ (48 % bezogen auf eingesetztes $C_{60}$) zurückgewonnen
536 mg (57 % bezogen auf eingesetzte CH-acide Verbindung)

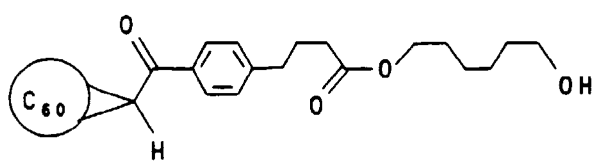

$R_f$ ($SiO_2$, $CH_2Cl_2$/MeBH 40/1) = 0.36
MS (FAB) : 1024 ($M^{\ominus}$, 50 %), 734 (35 %), 720 (100 %)
$^1$H-NMR (360) MHz, $CDCl_3$) : $\delta$ = 8.38 (d, J = 8.3 Hz, 2 H) 7.48 (d, J = 8.3 Hz, 2 H), 5.62 (s, 1 H), 4.08 (t, J = 6.7 Hz, 2 H), 3.63 (t, J = 6.5 Hz, 2 H), 2.81 (t, J = 7.6 Hz, 2 H), 2.37 (t, J = 7.4 Hz, 2 H), 2.04 (quint, J = 7.5 Hz, 2 H), 1.64 (m, 2 H), 1.57 (m, 2 H), 1.39 (m, 4 H)
$^{13}$C-NMR (90) MHz, $CDCl_3$) : $\delta$ = 189.2 (C=O), 173.2 (C=O), 149.0 (1C), 148.1, 146.7, 145.6, 145.4, 145.3, 145.2, 145.2, 145.1, 144.9 (1C), 144.7, 144.7, 144.6, 144.6, 144.4, 143.9, 143.7, 143.3, 143.2 (1C), 143.0, 143.0, 143.0, 142.8, 142.5, 142.3, 142.2, 142.1, 141.2, 141.0, 139.6, 136.6, 136.6, 134.0, 129.5, 129.2, 72.4, 64.5, 62.8, 44.2, 35.3, 33.6, 32.6, 28.7, 26.1, 25.8, 25.4.

Beispiel 22

247 mg (0.34 mmol) $C_{60}$ wurden in 100 ml Toluol mit 155 mg (0.33 mmol) 3-[4-(2-Bromacetyl)-phenyl]-1-(1,4,7,10-tetraoxa-13-aza-cyclodec-13-yl)-propan-1-on und 50 mg (0.33 mmol) DBU für 24 Stunden gerührt.
Chromatographie:

a) 35 g Kieselgel (0.063-0.2 mm); 100 ml Toluol, 315 ml Toluol/Ethanol 100:5, 55 ml Toluol/Ethanol 100:10, 100 ml Methylenchlorid/Ethanol 95:5
b) 25 g Kieselgel (0.063-0.2 mm); 133 ml Toluol/Ethanol 130:3, 132 ml Toluol/Ethanol 130:2, 105 ml Toluol/Ethanol

100:5, 52 ml

Methylenchlorid/Ethanol 50:2, 105 ml Methylenchlorid/Ethanol 100:5 154 mg (42 % bezogen auf eingesetzte CH-acide Verbindung)

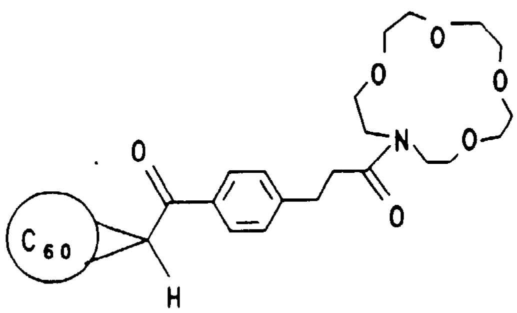

$R_f$ ($SiO_2$, Toluol/Methanol 20:1) = 0.21
MS (FAB) : 1112 ($MH^+$, 100 %), 720 (85 %)
$^1$H-NMR (360 MHz, $CD_2Cl_2$) $\delta$ = 8.40 (d, J = 8.3 Hz, 2 H), 7.56 (d, J = 8.2 Hz, 2 H), 5.72 (s, 1 H), 3.72 (t, J = 6.6Hz, 2 H), 3.64-3.53 (m, 16 H), 3.49 (t, J = 6.6 Hz, 2 H), 3.12 (t, J = 7.5 Hz, 2 H), 2.77 (t, J = 7.5 Hz, 2 H)
$^{13}$C-NMR (100 MHz, $CDCl_3$) $\delta$ = 189.20 (C=O), 171.99 (C =O), 149.22 (1C), 148.11, 146.76, 145.60, 145.41, 145.28, 145.19, 145.17, 145.07, 144.87 (1C), 144.73, 144.67, 144.65, 144.63, 144.62, 144.34, 143.94, 143.70, 143.33, 143.16 (1C), 143.04, 142.98 (1C), 142.97, 142.78, 142.49, 142.26, 142.23, 142.10, 141.20, 140.95, 139.55, 136.62, 133.92, 129.51, 129.20, 72.40, 71.70, 70.68, 70.40, 70.20, 70.16, 70.14, 69.75, 69.64, 50.52, 49.56, 44.29, 34.24, 31.50

Beispiel 23

940 mg (1.3 mmol) $C_{60}$ wurden in 380 ml Toluol mit 500 mg (1.27 mmol) 3-[4-(2-Bromacetyl)-phenyl]-propionsäure-14-nitrophenyl)ester und 194 mg (1.27 mmol) DBU für 20 Stunden gerührt.
Chromatographie: 180 g Kieselgel (0.063-0.2 mm); 1600 ml Toluol 333 mg $C_{60}$ (35 % bezogen auf eingesetztes $C_{60}$) 704 mg (53 % bezogen auf eingesetzte CH-acide Verbindung)

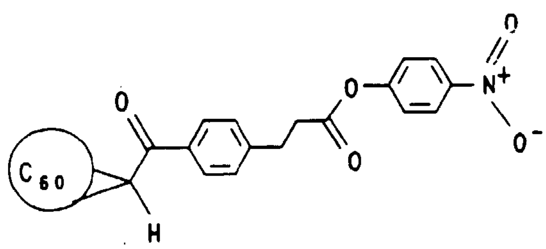

$R_f$ ($SiO_2$, Toluol) = 0.21
MS (FAB) : 1031 ($M^{\ominus}$, 50 %), 720 (100 %)
$^1$H (360 MHz, $CDCl_3$) $\delta$ = 8.43 (d, J = 8.3 Hz, 2 H), 8.25 (m, 2 H), 7.57 (d, J = 8.1 Hz, 2 H), 1 Signal für 2 aromatische Protonen werden von den Toluolprotonen verdeckt, 5.61 (s, 1 H), 3.24 (d, J = 7.3 Hz, 2 H), 3.04 (d, J = 7.3 Hz, 2 H)

Beispiel 24

1757 mg (2.44 mmol) $C_{60}$ wurden in 710 ml Toluol mit 823 mg (2.03 mmol) 4-[4-(2-Bromacetyl)-phenyl]-buttersäure-(4-nitrophenyl)ester und 310 mg (2.03 mmol) DBU für 7 Stunden gerührt.
Chromatographie: 400 g Kieselgel (0.063-0.2 mm); 3500 ml Toluol
1128 mg $C_{60}$ (53 % bezogen auf eingesetzte CH-acide Verbindung)

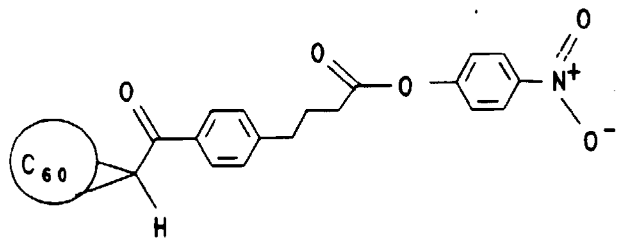

$R_f$ ($SiO_2$, Toluol) = 0.25
MS (FAB) : 1045 ($M^{\ominus}$, 20 %), 720 (100 %)
$^1$H-NMR (360 MHz, $CD_2Cl_2$) $\delta$ = 8.43 (d, J = 8.1 Hz, 2 H), 8.27 (d, J = 9.0 Hz, 2 H), 7.56 (d, J = 8.1 Hz, 2 H), 7.31 (d, J = 9.0 Hz, 2 H), 5.72 (s, 1 H), 2.93 (t, J = 7.6 Hz, 2 H), 2.71 (t, J = 7.4 Hz, 2 H), 2.19 (quint, 7.6 Hz, 2 H)
$^{13}$C-NMR (90 MHz, $CDCl_3$) $\delta$ = 189.2, 170.7, 155.3 (1C), 148.3 (1C), 148.0, 146.7, 145.6, 145.4, 145.3, 145.2, 145.2, 145.1, 144.9 (1C), 144.8, 144.7, 144.7, 144.6, 144.6, 144.4, 143.9, 143.7, 143.3, 143.2 (1C), 143.1, 143.0, 143.0, 142.8, 142.5, 142.3, 142.2, 142.1, 141.2, 141.0, 139.6, 136.6, 134.2 (1C), 129.3, 129.3, 125.3, 122.3, 72.3, 44.2, 35.2, 33.5, 25.8.

Beispiel 25

471 mg (0.65 mmol) $C_{60}$ wurden in 200 ml Toluol mit 168 mg (0.65 mmol) 3-[4-(2-Bromacetyl)-phenyl]-propan-1-ol und 100 mg (0.65 mmol) DBU für 17 Stunden gerührt.
Chromatographie:

a) 80 g Kieselgel (0.063-0.2 mm); 300 ml Toluol/Methanol 100:1, 300 ml Toluol/Methanol 30:1, 500 ml Toluol/Methanl 25:1, 200 ml Toluol/Methanol 20:1
b) 60 g Kieselgel (0.063-0.2 mm); 900 ml Methylenchlorid/Methanol 100:1 290 mg $C_{60}$ (53 % bezogen auf eingesetzte CH-acide Verbindung)

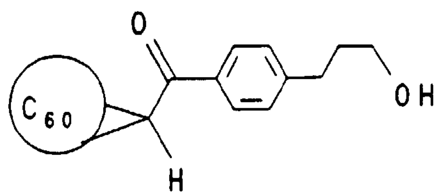

$R_f$ ($SiO_2$, $CH_2Cl_2$/MeOH 80:1) = 0.25
MS (FAB) : 896 ($M^{\ominus}$, 100 %), 720 (90 %)
$^1$H-NMR (360 MHz, $CDCl_3$) $\delta$ = 8.39 (d, J = 8.3 Hz, 2 H), 7.50 (d, J = 8.3 Hz, 2 H), 5.62 (s, 1 H), 3.73 (m, 2 H), 2.88 (t, J = 7.7 Hz, 2 H, 1.98 (m, 2 H)

Beispiel 26

495 mg (0.65 mmol) $C_{60}$ wurden in 200 ml Toluol mit 200 mg (0.58 mmol) 4-[4-(2-Brom-2-chloracetyl)phenyl] buttersäure-tert.-butylester und 94 mg (0.62 mmol) DBU für 17 Stunden gerührt.
Chromatographie: 200 g Kieselgel (0.063-0.2 mm); 1000 ml Toluol
180 mg $C_{60}$ (36 % bezogen auf eingesetztes $C_{60}$)
370 mg (62 % bezogen auf eingesetzte CH-acide Verbindung)

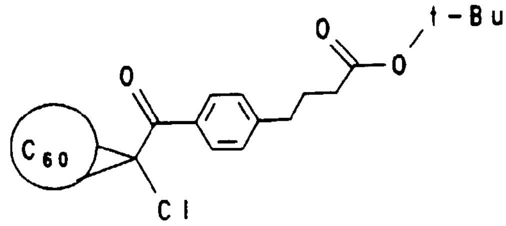

$R_f$ ($SiO_2$, Toluol) = 0.32
MS (FAB) : 1014 ($M^{\ominus}$, 100 %), 720 (50 %)

Beispiel 27

74 mg (0.073 mmol) des tert.-Butylesters aus Beispiel 26 wurden in 10 ml Methylenchlorid gelöst, und zu dieser Lösung wurden 148 mg (1.58 mmol) Methansulfonsäure gegeben. Nach 2 Minuten wurde 1 ml Wasser und 30 ml Methylenchlorid zugegeben. Die organische Phase wurde abgetrennt und mit Magnesiumsulfat getrocknet.
Chromatographie: 15 g Kieselgel (0.063-0.1 mm); 140 ml
Methylenchlorid/Essigsäure 40:1
37,7 mg (53 %)

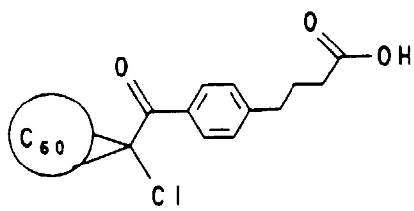

$R_f$ ($SiO_2$, $CH_2Cl_2$/ACOH 20:0.5) = 0.36
MS (FAB) : 958 ($M^{\ominus}$, 100 %), 720 (70 %)

Beispiel 28

100 mg (0.11 mmol) des Alkohols aus Beispiel 25 wurden in 30 ml Methylenchlorid suspendiert, 89 mg (0.89 mmol) Bernsteinsäureanhydrid und 27 mg (0.22 mmol) DMAP zugegeben und das Reaktionsgemisch 1 Woche bei Raumtemperatur gerührt. Ein Tropfen konz. Salzsäure wurde zu dem Reaktionsgemisch gegeben und dieses mit Magnesiumsulfat getrocknet.
Chromatographie: 50 g Kieselgel (0.063-0.2 mm); 800 ml
Methylenchlorid/Methanol 200:1, 800 ml Methylenchlorid 200:3
56 mg (50 %)

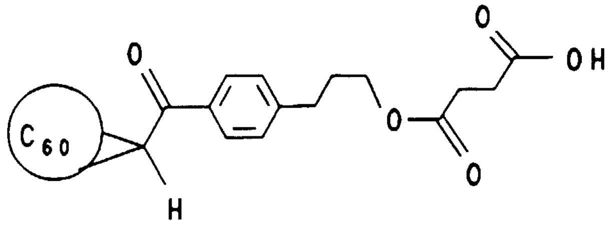

$R_f$ ($SiO_2$, $CH_2Cl_2$/MeOH 20:1) = 0.08
MS (FAB) : 997 ($MH^{\oplus}$, 25 %), 720 (100 %)

Beispiel 29

Eine Lösung von 19 mg (0.018 mmol) des p-Nitrophenylester aus Beispiel 23 in 18 ml Toluol wurde bei Raumtemperatur mit 2.0 mg (0.018 mmol) Benzylamin 12 Tage gerührt.
Chromatographie: 5 g Kieselgel (0.063-0.2 mm); 50 ml Methylenchlorid, 52 ml Methylenchlorid/Methanol 25:1
9,5 mg (51 %)

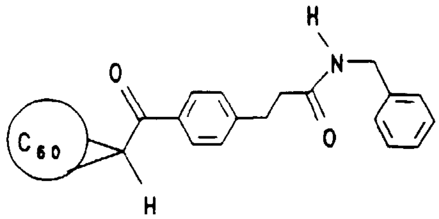

$R_f$ ($SiO_2$, $CH_2Cl_2$) = 0.07
MS (FAB) : 999 ($M^{\ominus}$, 70 %), 720 (100 %)

Beispiel 30

Eine Lösung von 50 mg (0.048 mmol) des p-Nitrophenylester aus Beispiel 23 in 75 ml Toluol wurde bei Raumtemperatur mit 20 mg (0.16 mmol) Glycinmethylester Hydrochlorid und 16 mg (0.16 mmol) Triethylamin 10 Tage gerührt. Das Reaktionsgemisch wurde filtriert und chromatographiert.
Chromatographie:

a) 30 g Kieselgel (0.063-0.2 mm); 101 ml Methylenchlorid/Methanol 100:1, 102 ml Methylenchlorid/Methanol 100:2, 103 ml Methylenchlorid/Methanol 100:3, 180 ml Methylenchlorid/Methanol/Triethylamin 20:1.5:1
b) 12 Kieselgel (0.063-0.2 mm); 123 ml Methylenchlorid/Methanol 100:2.5 35 mg (75 %)

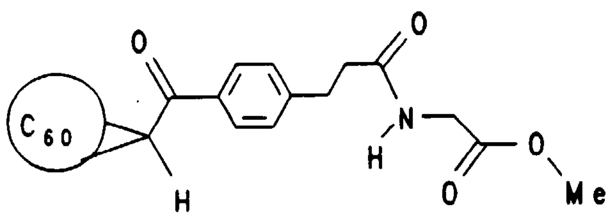

$R_f$ ($SiO_2$, $CH_2Cl_2$/MeOH 40:1) = 0.33
MS (FAB) : 981 ($M^{\ominus}$)
$^1$H-NMR (360 MHz, $CDCl_3$) $\delta$ = 8.38 (d, J = 8.3 Hz, 2 H), 7.51 (d, J = 8.3 Hz, 2 H), 5.61 (s, 1 H), 4.04 (m, 2 H), 3.75 (s, 3 H), 3.14 (t, J = 7.6 Hz, 2 H), 2.64 (t, J = 7.6 Hz, 2 H).

Beispiel 31

Eine Lösung von 100 mg (0.097 mmol) des p-Nitrophenylester aus Beispiel 23 in 40 ml Toluol wurde bei Raumtemperatur mit 9.9 mg (0.097 mmol) 1,3-Bis-(methylamino)-propan gerührt. Zu dem Reaktionsgemisch wurden 100 mg (1.8 mmol) Methylisocyanat gegeben und 6 Tage bei Raumtemperatur gerührt. Das Reaktionsgemisch wurde filtriert und chromatographiert.
Chromatographie:
a)50 g Kieselgel (0.063-0.2 mm); 410 ml Methylenchlorid/Methanol 200:5, 210 ml Methylenchlorid/Methanol 100:5
43 mg (42 %)

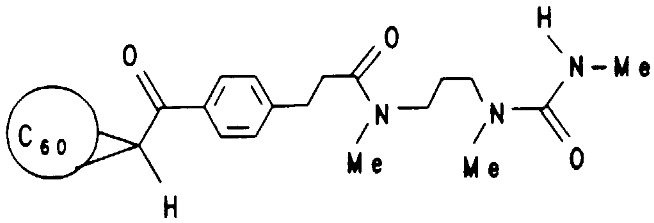

$R_f$ ($SiO_2$, $CH_2Cl_2$/MeOH 20:1) = 0.13
MS (FAB) : 1051 ($M^{\ominus}$)

Beispiel 32

Analog zu Beispiel 3 wurden 59 mg (0.082 mmol) $C_{60}$ in 25 ml Toluol mit 20.7 mg (0.163 mmol) Jod, 22.4 mg (0.082 mmol) Methylenbisphosphonsäure-tetraethylester und 24.9 mg DBU umgesetzt. Nach 3 Tagen Reaktionszeit bei Raumtemperatur wurde der Reaktionsansatz filtriert und an 30 g Kieselgel (0.063-0.2 mm) chromatographiert. $C_{60}$ wurde mit Toluol eluiert und das Monoadditionsprodukt mit 150 ml Methylenchlorid/Ethanol 20:1 eluiert. Es wurden 8 mg (9 % bezogen auf eingesetztes $C_{60}$) Monoadditionsprodukt

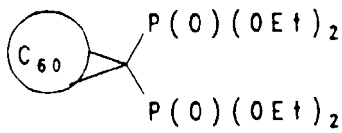

erhalten.

$R_f$ ($SiO_2$, $CH_2Cl_2$/EtBH 20:1) = 0.4
MS (FAB) : 1006 ($M^{\ominus}$, 50 %), 720 (100 %)

Beispiel 33

Analog zu Beispiel 3 wurden 59 mg (0.082 mmol) $C_{60}$ in 25 ml Toluol mit 20.7 mg (0.163 mmol) Jod, 8.1 mg (0.082 mmol) Acetylaceton und 24.9 mg DBU umgesetzt. Nach 3 Tagen Reaktionszeit bei Raumtemperatur wurde der Reaktionsansatz filtriert und an 40 g Kieselgel (0.063-0.2 mm) chromatogrpahiert. $C_{60}$ wurde mit 100 ml Toluol/i-Hexan 1:1 eluiert und das Monoadditionsprodukt mit 250 ml Toluol/i-Hexan 2:1 eluiert. Es wurden 12 mg (17 % bezogen auf eingesetztes $C_{60}$) Monoadditionsprodukt

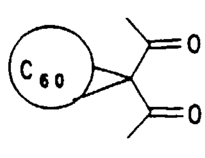

erhalten.

$R_f$ ($SiO_2$, Toluol) = 0.4
MS (FAB) : 818 ($M^{\ominus}$, 100 %), 720 (100 %)
$^1$H-NMR (360 MHz, $CDCl_3$): $\delta$ = 2.82 (s).

Beispiel 34

In einem evakuierten und mit Argon belüfteten 250 ml Stickstoffkolben wurden 251 mg (0.299 mmol) $C_{70}$ in 200

ml Toluol gegeben und zu der Suspension wurden 55 mg (0.230 mmol) Brommalonsäurediethylester und 35.8 mg (0.235 mmol) DBU gespritzt. Nach 4 h Rühren bei Raumtemperatur wurde die Suspension filtriert und die klare Lösung auf ca. 75 ml eingeengt. Laut HPLC wurden 60 % des eingesetzten $C_{70}$ umgesetzt. Nach Chromatographie an $SiO_2$ (0.063-0.2 mm) mit Toluol/i-Hexan und Toluol wurden 110 mg $C_{70}$ (43 % bezogen auf eingesetztes $C_{70}$) zurückgewonnen und

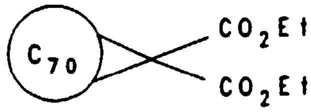

wurde in mikrokristalliner Form erhalten (138 mg, 60 % bezogen auf Brommalonat).

$R_f$ ($SiO_2$, Toluol) = 0.47
MS(FAB): 998 ($M^{\ominus}$)
$^1$H-NMR (360 MHz, $CDCl_3$): $\delta$ = 4.50 (m, 4 H), 1.46 (t, J = 7.1 Hz, 6 H).
$^{13}$C-NMR (100 MHz, $CDCl_3$): $\delta$ = 163.45, 155.12, 151.38 (3C), 151,19, 150.74, 150.60, 149.36, 149.27, 149.12, 148.72, 148.59, 148.53, 148.49, 147.67, 147.54, 147.32, 147.02, 146.47 (1C), 145.95, 145.93, 144.89, 143.96, 143.86, 143.54, 142.95, 142.85, 142.25, 141.68, 140.81, 136.98, 133.59, 132.84, 130.95, 130.91, 130.83, 66.90, 66.24, 63.47, 37.22, 14.23.

| | | | |
|---|---|---|---|
| Analyse | ber. für $C_{77}H_{10}O_4$: | C: 92.6 % | H: 1.0 % |
| | gef. | C: 93.7 % | H: 0.9 % |

Beispiel 35

Analog zu Beispiel 34 wurden 250 mg (0.3 mmol) $C_{70}$ in 250 ml Toluol mit 66 mg (0.31 mmol) Brommalonsäuredimethylester und 48 mg (0.31 mmol) DBU umgesetzt. Nach 3 h Rühren bei Raumtemperatur wurde die Suspension filtriert und die klare braunviolette Lösung auf ca. 60 ml eingeengt und an $SiO_2$ (0.063-0.2 mm) mit Toluol/i-Hexan 1:1 bis 3:1 und Toluol chromatographiert. Es wurden 42 mg $C_{70}$ (16.8 %) zurückgewonnen und

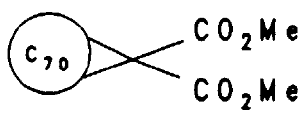

wurde in mikrokristalliner Form erhalten (137 mg, 47 %).

$R_f$ ($SiO_2$, Toluol) = 0.36
MS(FAB): 970 ($M^{\ominus}$, 70 %), 840 (100 %)
$^1$H-NMR (360 MHz, $CDCl_3/CS_2$): $\delta$ = 4.02 (s)
$^{13}$C-NMR (100 MHz, $CDCl_3/CS_2$): $\delta$ = 163.29, 154.77, 151.19, 151.12 (1C), 150.94, 150.48, 150.35, 149.12, 149.03, 148.91, 148.54, 148.37, 148.30, 148.26, 147.38, 147.30, 147.10, 146.78, 146.14 (1C), 145.77, 145.71, 144.63, 143.77, 143.66, 143.38, 142.67, 142.56, 142.05, 141.45, 140.62, 136.64, 133.36, 132.59, 130.73 (4C), 130.60, 53.72:,wegen schlechtem Signal-Rauschverhältnis: keine Signale für den Cyclopropanring.

Beispiel 36

Analog Beispiel 34 wurden 251 mg (0.299 mmol) $C_{70}$ in 250 ml Toluol mit 139 mg (0.3 mmol) Brommalonsäuredidecylester und 45.6 mg (0.3 mmol) DBU umgesetzt. Nach 4 h Reaktionszeit bei Raumtemperatur wurde der Reaktionsansatz filtriert, das Lösungsmittel entfernt und der Rückstand mit Diethylether extrahiert. Die Etherlösung wurde

durch eine kurze Kieselgelsäule filtriert und das Lösungsmittel entfernt.
Der Rückstand wurde in ca. 5 ml i-Hexan/Toluol 2:1 gelöst und an 250 g Kieselgel (0.063-0.2 mm) mit 2.2 l i-Hexan/Toluol 2:1 und 1.1 l i-Hexan/Toluol 1:2 chromatographiert.
129 mg (35 % bezogen auf eingesetztes $C_{70}$) Monoaddukt.

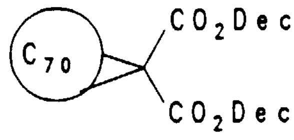

sowie 95 mg (19 %) Diaddukt als Isomerengemisch wurden erhalten.

$R_f(SiO_2$; Toluol/i-Hexan 1:2) = 0.18
MS(FAB): 1222 ($M^{\ominus}$, 60 %), 840 (100 %)
$^1$H-NMR (360 MHz, $CDCl_3$): $\delta$ = 4.42 (t, J= 6.5 Hz, 4H), 1.81 (m, 4H), 1.46 (m, 4H), 1.25 (m, 24H), 0.86 (s, 6H)
$^{13}$C-NMR (100 MHz, $CDCl_3$): $\delta$ = 163.51, 155.06, 151.35, 151.32(1C), 151.14, 150.69, 150.55, 149.31, 149.22, 149.08, 148.67, 148.53, 148.48, 148.45, 147.61, 147.49, 147.27, 146.97, 146.42(1C), 145.90, 145.88, 144.82, 143.91, 143.81, 143.49, 142.94, 142.79, 142.17, 141.62, 140.72, 136.98, 133.54, 132.79, 130.89, 130.85, 130.77, 67.52(2C), 66.91(1C), 66.24(1C), 37.39(1C), 31.89, 29.63, 29.57, 29.34, 29.25, 28.58, 26.02, 22.69, 14.12.

Beispiel 37

Analog zu Beispiel 34 wurden 250 mg (0.3 mmol) $C_{70}$ in 250 ml Toluol mit 139 mg (0.3 mmol) Brommalonsäuredi-(2-(2-Methoxyethoxy)-ethyl)ester und 45.6 mg(0.3 mmol) DBU umgesetzt. Nach 6 h Reaktionszeit bei Raumtemperatur (75 %iger Umsatz des eingesetzten $C_{70}$ laut HPLC) wurde der Reaktionsansatz filtriert und an 45 g Kieselgel (0.063-0.2 mm) chromatographiert. $C_{70}$ wurde mit Toluol eluiert und die Additionsprodukte mit 800 ml Toluol/Diethylether 1:1 und 400 ml Toluol/Ethanol 9:1 chromatographiert. Es wurden 75 mg (14 %) $C_{70}$, 139 mg (40 %) Monoaddukt

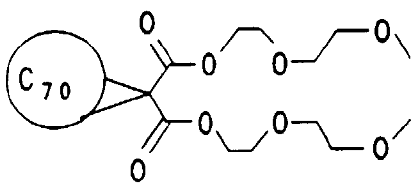

und 96 mg (22 %) Diaddukte als Isomerengemisch erhalten.

$R_f(SiO_2$; Toluol/Diethylether 1:1) = 0.25
MS(FAB): 1146 ($M^{\ominus}$, 55 %), 840 (100 %)
$^1$H-NMR (360 MHz, $CDCl_3$): $\delta$ = 4.59 (m, 4H), 3.85 (m, 4H), 3.68 (m, 4H), 3.54 (m, 4H), 3.37 (s, 6H).

Beispiel 38

Analog zu Beispiel 34 wurden 251 mg (0.3 mmol) $C_{70}$ in 250 ml Toluol suspendiert und mit 75 mg (0.45 mmol) 2-Chloracetessigsäureethylester und 60 mg (0.39 mmol) DBU umgesetzt. Nach 24 h Reaktionszeit bei Raumtemperatur wurde das Reaktionsgemisch filtriert, der Umsatz laut HPLC zu 64 % bestimmt und die Lösung auf ca. 60 ml eingeengt. Chromatographie an $SiO_2$ (0.063-0.2 mm) mit Toluol/i-Hexan 1:1 bis 4:1 lieferte 104 mg (35.7 %)

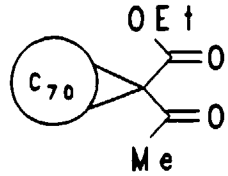

$R_f$ ($SiO_2$, Toluol) = 0.38.
MS(FAB): 968 ($M^-$).
$^1H$-NMR (360 MHz, $CDCl_3$): $\delta$ = 4.52 (m, 2H), 2.82 (s, 3H), 1.48 (t, J= 7.1 Hz, 3H).

Beispiel 39

Analog zu Beispiel 34 wurden 250 mg (0.3 mmol) $C_{70}$ in 250 ml Toluol mit 75 mg (0.37 mmol) ω-Bromacetophenon und 50.2 mg (0.37 mmol) DBU umgesetzt. Nach 22 h Reaktionszeit bei Raumtemperatur (56 %iger Umsatz des eingesetzten $C_{70}$ laut HPLC) wurde der Reaktionsanstz filtriert, und die Reaktionslösung wurde auf 70 ml eingeengt. Nach dreimaliger Chromatographie an 250 g Kieselgel (0.063-0.2 mm) mit Toluol/i-Hexan 1:3, 1:2 und 1:1 wurden 58.2 mg (20 %) Monoaddukt

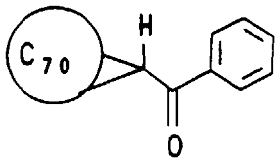

isoliert.

$R_f$($SiO_2$; Toluol/i-Hexan 1:2) = 0.12
MS(FAB): 958 ($M^-$, 70 %), 840 (100 %)
$^1H$-NMR (360 MHz, $CDCl_3$): $\delta$ = 8.42 (m, 2 $H_{ortho}$) 7.76 (m, 1 $H_{para}$), 7.68 (m, 2 $H_{meta}$), 4.42 (s, 3H).

Beispiel 40

Analog zu Beispiel 34 wurden 200 mg (0.24 mmol) $C_{70}$ in 200 ml Toluol mit 62.5 g (0.2 mmol) 4-[4-(2-Brom-acetyl)-phenyl]-buttersäure-ethylester und 30.5 mg (0.2 mmol) DBU umgesetzt. Nach 24 h Reaktionszeit bei Raumtemperatur wurden 3 Tropfen 1 molare Schwefelsäure zugesetzt, mit Magnesiumsulfat getrocknet und filtriert. Die Lösung wurde auf 80 ml eingeengt und an 80 g Kieselgel (0.063-0.2 mm) mit 600 ml Toluol chromatographiert. Es wurden 110 mg (55 % bezogen auf eingesetztes $C_{70}$) $C_{70}$ und 34 mg (16 % bezogen auf eingesetztes $C_{70}$ und 29 % bezogen auf umgesetzes $C_{70}$) Monoaddukt

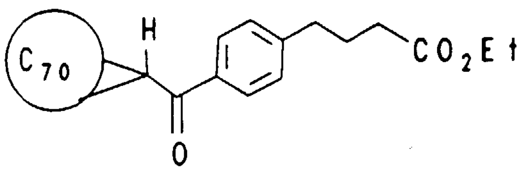

isoliert.

$R_f$($SiO_2$; Toluol) = 0.27
MS(FAB): 1072 ($M^{\ominus}$, 50 %), 840 (100 %).
$^1H$-NMR (360 MHz, $CD_2Cl_2$): $\delta$ = 8.36 (d, J= 8.3 Hz, 2H), 7.52 (d, J= 8.3 Hz, 2H), 4.50 (s, 1H), 4.13 (m, 2H), 2.84

(t, J= 7.7 Hz, 2H), 2.39 (t, J= 7.4 Hz, 2H), 2.04 (quint, J= 7.5 Hz, 2H), 1.26 (t, J= 7.1 Hz, 3H).

## Patentansprüche

**1.** Fullerenderivate der Formel I

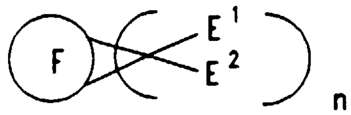

wobei die Symbole und Indices die folgende Bedeutung haben:

F: ein Fullerenrest der Formel $C_{(20+2m)}$ mit m = 20, 25, 28, 29;

$E^1$, $E^2$: gleich oder verschieden COOH, COOR, $CONRR^1$, CHO, COR, CN, $P(O)(OR)_2$ und $SO_2R$, wobei R, $R^1$ einen geradkettigen oder verzweigten, gegebenenfalls ein- oder mehrfach gleich oder verschieden substituierten aliphatischen Rest ($C_1$ bis $C_{20}$) darstellen, in dem bis zu jede dritte $CH_2$-Einheit durch O oder $NR^4$ ersetzt sein kann, mit $R^4$ = ($C_1$-$C_{20}$)-Alkyl oder Benzyl, oder einen Benzylrest oder Phenylrest bedeuten, die gegebenenfalls durch 1 bis 5 Substituenten R, OH, OR, COOR, OCOR, $SO_3H$, $SO_2Cl$, F, Cl, Br, $NO_2$ und CN substituiert sein können,

oder gleich

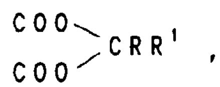

oder verschieden voneinander COR, R, oder H,
oder verschieden voneinander COR/R oder F/Cl/Br, wobei R die obengenannte Bedeutung hat,
oder verschieden voneinander $NO_2$, $R^3$ oder H, wobei $R^3$ einen gegebenenfalls ein- oder mehrfach substituierten aliphatischen Rest ($C_1$ bis $C_{20}$) darstellt;

n: eine natürliche Zahl von 1 bis 10+m mit m= 20, 25, 28, 29.

**2.** Fullerenderivat der Formel 1 nach Anspruch 1, wobei die Symbole und Indices folgende Bedeutung haben:

F: ein Fullerenrest der Formel $C_{(20+2m)}$ mit m = 20, 25, 28, 29,

$E^1$, $E^2$: gleich oder verschieden CCOR, COR, $P(O)(OR)_2$, COOH, CN, wobei R einen geradkettigen oder verzweigten, gegebenenfalls ein- oder mehrfach gleich oder verschieden substituierten aliphatischen Rest ($C_1$ bis $C_{20}$) darstellt, in dem bis zu jede dritte $CH_2$-Einheit durch O oder $NR^4$ ersetzt sein kann, mit $R^4$ = ($C_1$-$C_{20}$)-Alkyl oder Benzyl, oder einen Benzylrest oder Phenylrest bedeutet, die gegebenenfalls durch 1 bis 3 Substituenten R, OH, OR, COOR, OCOR, $SO_3H$, $SO_2Cl$, F, Cl, Br, $NO_2$ und CN substituiert sein können,

oder gleich

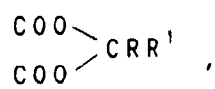

oder verschieden voneinander COR, R oder H,
oder verschieden voneinander COR/R oder F/Cl/Br,

n: eine natürliche Zahl von 1-12.

**3.** Fullerenderivate der Formel I nach Anspruch 1, wobei die Symbole und Indices folgende Bedeutung haben:

F : $C_{60}$, $C_{70}$
E1 / E2: $CO_2R^1$ / $CO_2R^2$ ;
$CO_2R^1$ / $COR^2$ ;
$CO_2R^1$ / CN;
COAr / $R^1$ oder H;
COAr / $R^1$ oder Cl;

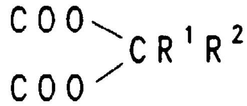 ;

$COR^1$ / $COR^2$;
$P(O)(OR^1)_2$ / $P(O)(OR^2)_2$;
COOH / COOH;
wobei $R^1$ und $R^2$, gleich oder verschieden, einen geradkettigen oder verzweigten, gegebenenfalls ein- oder mehrfach gleich oder verschieden substituierten Alkylrest ($C_1$ bis $C_{20}$) darstellen, in dem bis zu jede dritte $CH_2$-Einheit durch O oder $NR^4$ ersetzt sein kann, mit $R^4$ = ($C_1$-$C_{20}$)-Alkyl oder Benzyl, oder einen Benzylrest oder Phenylrest bedeutet, die gegebenenfalls durch 1 bis 3 Substituenten OH, OMe, $CO_2R^1$, $OOCR^1$, $SO_3H$, $SO_2Cl$, F, Cl, Br, $NO_2$ und CN substituiert sein können, und Ar einen Phenylrest darstellt, der ebenfalls durch 1 bis 3 Substituenten OH, OMe, Me, $CO_2R^1$, $OCOR^1$, $SO_3H$, $SO_2Cl$, F, Cl, Br, $NO_2$ und CN substituiert sein kann oder durch einen geradkettigen oder verzweigten, gegebenenfalls ein- oder zweifach gleich oder verschieden durch $COOR^5$, $CONHR^5$, $CONR^5_2$, $CONH_2$, $CONR^6$, COOH, OH oder $OCOR^5$, COOAr, $COOCH_2Ar$ substituierten aliphatischen Rest ($C_1$-$C_{20}$), vorzugsweise $C_1$-$C_{10}$, substituiert sein kann
mit $R^5$ =

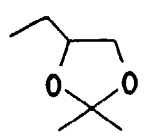 , 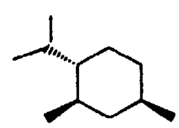 ,

$C_1$-$C_6$-Alkyl, Hydroxy-($C_1$-$C_6$)-Alkyl, Carboxy($C_1$-$C_6$)-alkyl oder ($C_1$-$C_3$)-Alkylcarboxyl($C_1$-$C_6$)-alkyl;

$R^6$ = $C_{11}$-$C_{17}$-Alkylen, in dem bis zu jede 3. $CH_2$-Einheit durch O ersetzt sein kann, das gemeinsam mit dem Amidstickstoff einen $C_{12}$-$C_{18}$Ring bildet,

und Ar wie oben definiert;
n : eine natürliche Zahl von 1-6 bedeutet.

**4.** Fullerenderivat der Formel I nach Anspruch 1, wobei die Symbole und Indices folgende Bedeutung haben:

F : $C_{60}$, $C_{70}$
$E^1$ / $E^2$: $CO_2Alkyl^1$ / $CO_2Alkyl^1$ ;
$CO_2Alkyl^1$ / $COAlkyl^2$ ;
COAr / Ar ;
COAr /$Alkyl^1$ ;
COAr / H
wobei $Alkyl^1$, $Alkyl^2$ einen geradkettigen oder verzweigten Alkylrest mit 1 bis 10 C-Atomen darstellt, in dem bis zu jede dritte $CH_2$-Einheit durch O ersetzt sein kann, und Ar eine Phenylgruppe bedeutet, die durch einen geradkettigen oder verzweigten, gegebenenfalls ein- oder zweifach gleich oder verschie-

den durch $COOR^5$, $CONHR^5_2$, $CONR^5{}_2$, $CONR^6$, COOH, OH oder $OCOR^5$ substituierten aliphatischen Rest ($C_1$-$C_6$) substituiert sein kann, worin $R^5$ und $R^6$ die obengenannte Bedeutung haben,

n : 1 oder 2.

**5.** Verfahren zur Herstellung von Fullerenderivaten gemäß Anspruch 1, dadurch gekennzeichnet, daß ein Fulleren der allgemeinen Formel $C_{(20+2m)}$ (m = natürliche Zahl) in einem aprotischen organischen Lösungsmittel, mit einer, CH-aciden Komponente der Formel II

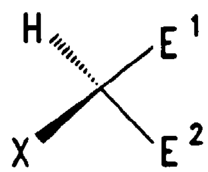

II

in der die Symbole und Indices folgende Bedeutung haben

F einen Fullerenrest der Formel $C_{(20+2m)}$ mit m = 20, 25, 28, 29,

$E^1$ und $E^2$ gleich oder verschieden COOH, COOR, $CONRR^1$, CHO, COR, CN, $P(O)(OR)_2$ oder $SO_2R$, wobei R, $R^1$ einen geradkettigen oder verzweigten, gegebenenfalls ein- oder mehrfach gleich oder verschieden substituierten aliphatischen Rest ($C_1$ bis $C_{20}$) darstellt, in dem bis zu jede dritte $CH_2$-Einheit durch O oder $NR^4$ ersetzt sein kann, mit $R^4$ = ($C_1$-$C_{20}$)-Alkyl oder Benzyl, oder einen Benzylrest oder Phenylrest bedeutet, gegebenenfalls durch 1 bis 5 Substitutenten R, OH, OR, COOR, OCOR, $SO_3H$, $SO_2Cl$, F, Cl, Br, $NO_2$ und CN substituiert sein können,

oder gleich

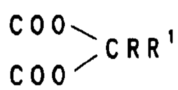

,

oder verschieden voneinander COR, R oder H,
oder verschieden voneinander COR/R oder F/Cl/Br,
oder verschieden voneinander $NO_2$, $R^3$ oder H, wobei $R^3$ ein gegebenenfalls ein- oder mehrfach substituierter aliphatischer Rest ($C_1$ bis $C_{20}$) sein kann,

X -Cl, -Br, -I, $-OSO_2Ar$, $OSO_2CF_3$, $OSO_2C_4F_9$

und Alkalimetallhydriden, Alkalimetallhydroxiden, Alkoholaten, Amiden, Aminen oder Guanidinen in einem Temperaturbereich von -78°C bis 180°C umgesetzt wird.

**6.** Verwendung von Fullerenderivaten nach einem oder mehreren der Ansprüche 1 bis 4 in optoelektronischen Bauelementen.

## Claims

**1.** A fullerene derivative of the formula I

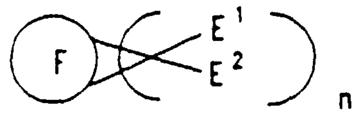

I

where the symbols and indices have the following meanings:

F: is a fullerene radical of the formula $C_{(20+2m)}$ where m = 20, 25, 28, 29;

$E^1$, $E^2$: are identical or different and are each COOH, COOR, $CONRR^1$, CHO, COR, CN, $P(O)(OR)_2$ and $SO_2R$, where R, $R^1$ are each a straight-chain or branched aliphatic radical ($C_1$ to $C_{20}$) which may be unsubstituted, monosubstituted or polysubstituted by identical or different substituents, in which radical up to every third $CH_2$ unit can be replaced by O or $NR^4$, where $R^4$ = ($C_1$-$C_{20}$)-alkyl or benzyl, or a benzyl radical or phenyl radical which can be unsubstituted or substituted by from 1 to 5 substituents R, OH, OR, COOR, OCOR, $SO_3H$, $SO_2Cl$, F, Cl, Br, $NO_2$ and CN

or together are

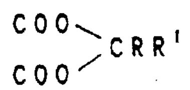

,

or are different from one another and are each COR, R or H,
or are different from one another and are each COR/R or F/Cl/Br, where R is as defined above,
or are different from one another and are each $NO_2$, $R^3$ or H, where $R^3$ is an unsubstituted, monosubstituted or polysubstituted aliphatic radical ($C_1$ to $C_{20}$);

n: is a natural number from 1 to 10 + m where m = 20, 25, 28, 29.

**2.** A fullerene derivative of the formula I as claimed in claim 1, where the symbols and indices have the following meanings:

F: is a fullerene radical of the formula $C_{(20+2m)}$ where m = 20, 25, 28, 29,

$E^1$, $E^2$: are identical or different and are each COOR, COR, $P(O)(OR)_2$, COOH, CN, where R is a straight-chain or branched aliphatic radical ($C_1$ to $C_{20}$) which may be unsubstituted, monosubstituted or polysubstituted by identical or different substituents, in which radical up to every third $CH_2$ unit can be replaced by O or $NR^4$, where $R^4$ = ($C_1$-$C_{20}$)-alkyl or benzyl, or a benzyl radical or phenyl radical which can be unsubstituted or substituted by from 1 to 3 substituents R, OH, OR, COOR, OCOR, $SO_3H$, $SO_2Cl$, F, Cl, Br, $NO_2$ and CN,

or together are

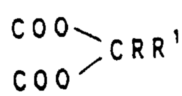

,

or are different from one another and are each COR, R or H,
or are different from one another and are each COR/R or F/Cl/Br,

n: is a natural number from 1 to 12.

**3.** A fullerene derivative of the formula I as claimed in claim 1, where the symbols and indices have the following meanings:

F : is $C_{60}$, $C_{70}$

E1/E2: are $CO_2R^1$ / $CO_2R^2$ ;
$CO_2R^1$ / $COR^2$ ;
$CO_2R^1$ / CN ;
COAr / $R^1$ or H;
COAr / $R^1$ or Cl;

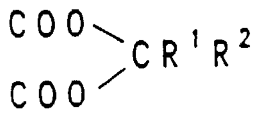 ;

$COR^1$ /$COR^2$;
$P(O)(OR^1)_2$ / $P(O)(OR^2)_2$;
COOH/COOH;
where $R^1$ and $R^2$ are identical or different and are each a straight-chain or branched alkyl radical ($C_1$ to $C_{20}$) which may be unsubstituted, monosubstituted or polysubstituted by identical or different substituents, in which radical up to every third $CH_2$ unit can be replaced by O or $NR^4$, where $R^4$ = ($C_1$-$C_{20}$)-alkyl or benzyl, or a benzyl radical or phenyl radical which can be unsubstituted or substituted by from 1 to 3 substituents OH, OMe, $CO_2R^1$, $OCOR^1$, $SO_3H$, $SO_2Cl$, F, Cl, Br, $NO_2$ and CN, and Ar is a phenyl radical which can likewise be substituted by from 1 to 3 substituents OH, OMe, Me, $CO_2R^1$, $OCOR^1$, $SO_3H$, $SO_2Cl$, F, Cl, Br, $NO_2$ and CN or can be substituted by a straight-chain or branched aliphatic radical ($C_1$-$C_{20}$), preferably $C_1$-$C_{10}$, which may be unsubstituted, monosubstituted or disubstituted by identical or different substituents $COOR^5$, $CONHR^5$, $CONR^5_2$, $CONH_2$, $CONR^6$, COOH, OH or $OCOR^5$, COOAr, $COOCH_2Ar$,
where $R^5$ =

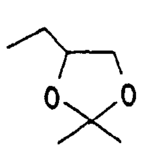 , 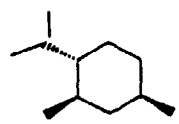 ,

$C_1$-$C_6$-alkyl, hydroxy-($C_1$-$C_6$)-alkyl, carboxy($C_1$-$C_6$)-alkyl or ($C_1$-$C_3$)-alkyl-carboxyl($C_1$-$C_6$)-alkyl;

$R^6$ = $C_{11}$-$C_{17}$-alkylene in which up to every 3rd $CH_2$ unit can be replaced by O and which together with the amide nitrogen forms a $C_{12}$-$C_{18}$ ring,

and Ar is as defined above;
n: is a natural number from 1 to 6.

**4.** A fullerene derivative of the formula I as claimed in claim 1, where the symbols and indices have the following meanings:

F : is $C_{60}$, $C_{70}$
$E^1/E^2$: are $CO_2Alkyl^1/CO_2Alkyl^1$;
$CO_2Alkyl^1/COAlkyl^2$;
COAr/Ar;
COAr/$Alkyl^1$;
COAr/H
where $Alkyl^1$, $Alkyl^2$ are each a straight-chain or branched alkyl radical having from 1 to 10 carbon atoms in which up to every third $CH_2$ unit can be replaced by O, and Ar is a phenyl group which can be substituted by a straight-chain or branched aliphatic radical ($C_1$-$C_6$) which may be unsubstituted, monosubstituted or disubstituted by identical or different substituents $COOR^5$, $CONHR^5_2$, $CONR^5_2$, $CONR^6$, COOH, OH or $OCOR^5$, where $R^5$ and $R^6$ are as defined above,
n: is 1 or 2.

**5.** A process for preparing fullerene derivatives as claimed in claim 1, which comprises reacting a fullerene of the

formula $C_{(20+2m)}$ (m = a natural number) in an aprotic organic solvent with a CH acid component of the formula II

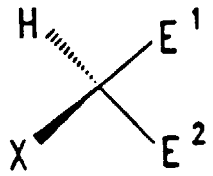

II

where the symbols and indices have the following meanings

F	is a fullerene radical of the formula $C_{(20+2m)}$ where m = 20, 25, 28, 29,

$E^1$ and $E^2$	are identical or different and are each COOH, COOR, $CONRR^1$, CHO, COR, CN, $P(O)(OR)_2$ or $SO_2R$, where R, $R^1$ are each a straight-chain or branched aliphatic radical ($C_1$ to $C_{20}$) which may be unsubstituted, monosubstituted or polysubstituted by identical or different substituents, in which radical up to every third $CH_2$ unit can be replaced by O or $NR^4$, where $R^4$ = ($C_1$-$C_{20}$)-alkyl or benzyl, or a benzyl radical or phenyl radical which can be unsubstituted or substituted by from 1 to 5 substituents R, OH, OR, COOR, OCOR, $SO_3H$, $SO_2Cl$, F, Cl, Br, $NO_2$ and CN

or together are

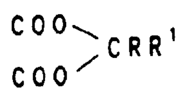

,

or are different from one another and are each COR, R or H,
or are different from one another and are each COR/R or F/Cl/Br,
or are different from one another and are each $NO_2$, $R^3$ or H, where $R^3$ can be an unsubstituted, monosubstituted or polysubstituted aliphatic radical ($C_1$ to $C_{20}$),

x	is -Cl, -Br, -I, $-OSO_2Ar$, $OSO_2CF_3$, $OSO_2C_4F_9$, and alkali metal hydrides, alkali metal hydroxides, alkoxides, amides, amines or guanidines in a temperature range from -78°C to 180°C.

**6.** Use of fullerene derivatives as claimed in one or more of claims 1 to 4 in optoelectronic components.

## Revendications

**1.** Dérivés de fullerènes de formule I

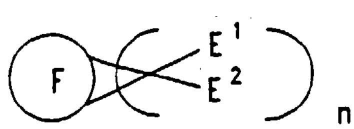

les symboles et les indices ayant les significations suivantes :

F :	un reste fullerène de formule $C_{20+2m}$ avec m = 20, 25, 28, 29 ;

$E^1$, $E^2$ :	identiques ou différents, représentent COOH, COOR, $CONRR^1$, CHO, COR, CN, $P(O)(OR)_2$ et $SO_2R$, où R, $R^1$ représentent un reste en $C_1$-$C_{20}$ aliphatique, linéaire ou ramifié, éventuellement substitué une ou plusieurs fois par des substituants identiques ou différents, dans lequel jusqu'à un sur trois motifs $CH_2$- peuvent être remplacés par O ou $NR^4$, avec $R^4$ = alkyle en $C_1$-$C_{20}$ ou benzyle, ou représentent un reste benzyle ou un reste phényle, qui peuvent être substitués éventuellement par 1 à 5 substituants, R, OH, OR, COOR, OCOR, $SO_3H$, $SO_2Cl$, F, Cl, Br, $NO_2$ et CN

ou sont

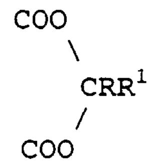

ou différents l'un de l'autre représentent COR, R, ou H,
ou différents l'un de l'autre représentent COR/R ou F/Cl/Br, R ayant la signification donnée ci-dessus,
ou différents l'un de l'autre représentent $NO_2$, $R^3$ ou H, $R^3$ étant un reste aliphatique en $C_1$-$C_{20}$ éventuellement une ou plusieurs fois substitué ;

n : est un entier naturel de 1 à 10+m avec m = 20, 25, 28, 29.

**2.** Dérivé de fullerène de formule I selon la revendication 1, les indices et les symboles ayant les significations suivantes :

F : représente un reste fullerène de formule $C_{20+m}$ avec m = 20, 25, 28, 29,
$E^1$, $E^2$ : identiques ou différents représentent COOR, COR, $P(O)(OR)_2$, COOH, CN, R étant un reste en $C_1$-$C_{20}$ aliphatique, linéaire ou ramifié, éventuellement substitué une ou plusieurs fois par des substituants identiques ou différents, dans lequel jusqu'à un sur trois motifs $CH_2$- pouvant être remplacés par O ou $NR^4$, avec $R^4$ = alkyle en $C_1$-$C_{20}$ ou benzyle, ou représentent un reste benzyle ou un reste phényle, qui peuvent être éventuellement substitués par 1 à 3 substituants R, OH, OR, COOR, OCOR, $SO_3H$, $SO_2Cl$, F, Cl, Br, $NO_2$ et CN, ou sont

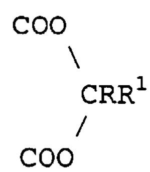

ou différents représentent COR, R ou H, ou différents l'un de l'autre représentent COR/R ou F/Cl/Br,
n : est un entier naturel de 1 à 12.

**3.** Dérivés de fullerène de formule I selon la revendication 1, les symboles et les indices ayant les significations suivantes :

F : $C_{60}$, $C_{70}$
$E^1$, $E^2$ : $CO_2R^1/CO_2R^2$ ;
$CO_2R^1/COR^2$ ;
$CO_2R^1/CN$ ;
$COAr/R^1$ ou H ;
$COAr/R^1$ ou Cl ;

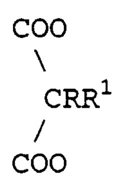

$COR^1/COR^2$ ;

$P(O)(OR^1)_2/P(O)(OR^2)_2$ ;
COOH/COOH ;
$R^1$ et $R^2$, identiques ou différents, représentent un reste alkyle en $C_1$-$C_{20}$ linéaire ou ramifié, éventuellement substitué une ou plusieurs fois par des substituants identiques ou différents, dans lequel jusqu'à un sur trois motifs $CH_2$- peuvent être remplacés par O ou $NR^4$, avec $R^4$ = alkyle en $C_1$-$C_{20}$ ou benzyle, ou représente un reste benzyle ou un reste phényle, qui peuvent être éventuellement substitués par 1 à 3 substituants OH, OMe, $CO_2R^1$, $OOCR^1$, $SO_3H$, $SO_2Cl$, F, Cl, Br, $NO_2$ et CN, et Ar représente un reste phényle, lequel peut être également substitué par 1 à 3 substituants OH, OMe, Me, $CO_2R^1$, $OCOR^1$, $SO_3H$, $SO_2Cl$, F, Cl, Br, $NO_2$ et CN, ou peut être substitué par un reste aliphatique en $C_1$-$C_{20}$, de préférence en $C_1$-$C_{10}$, linéaire ou ramifié, éventuellement substitué une ou deux fois par des substituants identiques ou différents $COOR^5$, $CONHR^5$, $CONR_2{}^5$, $CONH_2$, $C0NR^6$, COOH, OH ou $OCOR^5$, COOAr, $COOCH_2Ar$, avec $R^5$ =

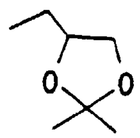
,
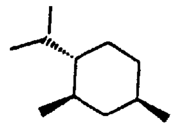

alkyle en $C_1$-$C_6$, hydroxyalkyle en $C_1$-$C_6$, carboxyalkyle en $C_1$-$C_6$, ou (alkyle en $C_1$-$C_3$)-carboxyalkyle en $C_1$-$C_6$ ;

$R^6$ = alkylène en $C_{11}$-$C_{17}$, dans lequel jusqu'à 1 sur 3 motifs $CH_2$- peuvent être remplacés par O, qui forme ensemble avec l'azote d'amide un cycle en $C_{12}$-$C_{18}$,

et Ar étant défini ci-dessus ;
n : est un entier naturel de 1 à 6.

**4.** Dérivé de fullerène de formule I selon la revendication 1, les indices et les symboles ayant les significations suivantes :

F : $C_{60}$, $C_{70}$
$E^1/E^2$ : $CO_2alkyle^1/CO_2alkyle^1$ ;
$CO_2alkyle^1/CO_2alkyle^2$ ;
COAr/Ar ;
COAr/alkyle$^1$ ;
COAr/H
où alkyle$^1$, alkyle$^2$ représentent un reste alkyle avec 1 à 10 atomes de carbone linéaires ou ramifiés, dans lequel jusqu'à 1 sur 3 motifs $CH_2$- peuvent être remplacés par O, et Ar représente un groupe phényle, qui peut être substitué par un reste aliphatique en $C_1$-$C_6$ linéaire ou ramifié, éventuellement une ou deux fois substitué par des substituants identiques ou différents $COOR^5$, $CONHR^5$, $CON_2{}^5$, $CONR^6$, COOH, OH ou $OCOR^5$, dans lequel $R^5$ et $R^6$ ont les significations données ci-dessus,
n : vaut 1 ou 2.

**5.** Procédé pour la préparation de dérivés de fullerènes selon la revendication 1, caractérisé en ce que l'on fait réagir un fullerène de formule générale $C_{20+2m}$ (m = entier naturel) dans un solvant organique aprotique avec un composé CH acide de formule II

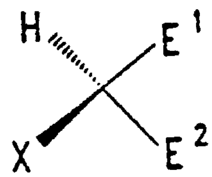
II

dans laquelle les symboles et les indices ont les significations suivantes :

F représente un reste fullerène de formule $C_{20+2m}$ avec m = 20, 25, 28, 29,

$E^1$ et $E^2$, identiques ou différents, représentent COOH, COOR, $CONRR^1$, CHO, COR, CN, $P(O)(OR)_2$ ou $SO_2R$, où R, $R^1$ représentent un reste aliphatique en $C_1$-$C_{20}$, linéaire ou ramifié, éventuellement substitué une ou plusieurs fois par des substituants identiques ou différents, dans lequel jusqu'à un sur trois motifs $CH_2$- peuvent être remplacés par O ou $NR^4$, avec $R^4$ = alkyle en $C_1$-$C_{20}$ ou benzyle, ou représente un reste benzyle ou un reste phényle, pouvant être éventuellement substitués par 1 à 5 substituants R, OH, OR, COOR, OCOR, $SO_3H$, $SO_2Cl$, F, Cl, Br, $NO_2$ et CN, ou représentent

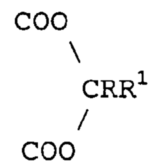

ou différents l'un de l'autre représentent COR, R ou H,
ou différents l'un de l'autre représentent COR/R ou F/Cl/Br,
ou différents l'un de l'autre représentent $NO_2$, $R^3$ ou H, $R^3$ pouvant être un reste aliphatique en $C_1$-$C_{20}$ éventuellement une ou plusieurs fois substitué,

X représente -Cl, -Br, -I, $-OSO_2Ar$, $OSO_2CF_3$, $OSO_2C_4F_9$, et des hydrures de métaux alcalins, des hydroxydes de métaux alcalins, des alcoolates, des amides, des amines ou des guanidines dans un intervalle de températures de -78 °C à 180 °C.

**6.** Utilisation de dérivés de fullerènes selon une ou plusieurs des revendications 1 à 4 dans des composants optoélectroniques.